(19) Europäisches Patentamt
European Patent Office
Office européen des brevets

(11) Veröffentlichungsnummer: **0 114 029**
**B1**

(12) **EUROPÄISCHE PATENTSCHRIFT**

(45) Veröffentlichungstag der Patentschrift: **16.09.87**

(21) Anmeldenummer: **83810584.9**

(22) Anmeldetag: **12.12.83**

(51) Int. Cl.⁴: **G 03 C 7/26** // C07D211/46, C07D211/58, C07D493/10, C07D471/10, C07D407/12, C07D401/12, C07D211/14

(54) **Farbphotographisches Aufzeichnungsmaterial.**

(30) Priorität: **16.12.82 CH 7316/82**

(43) Veröffentlichungstag der Anmeldung: **25.07.84 Patentblatt 84/30**

(45) Bekanntmachung des Hinweises auf die Patenterteilung: **16.09.87 Patentblatt 87/38**

(84) Benannte Vertragsstaaten: **BE DE FR GB IT**

(56) Entgegenhaltungen:
**EP-A-0 010 516**
**EP-A-0 011 051**
**FR-A-2 334 672**

(73) Patentinhaber: **CIBA-GEIGY AG, Klybeckstrasse 141, CH-4002 Basel (CH)**

(72) Erfinder: **Leppard, David G., Dr., Route de Bourguillon 6, CH-1723 Marly (CH)**
Erfinder: **Rody, Jean, Dr., Rütiring 82, CH-4125 Riehen (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die vorliegende Erfindung betrifft ein farbphotographisches Aufzeichnungsmaterial, das in mindestens einer lichtempfindlichen Silberhalogenidemulsionsschicht und/oder in mindestens einer der üblichen Hilfsschichten als Stabilisator mindestens eine spezifische Polyalkylpiperidin-Verbindung enthält.

Polyalkylpiperidine sind als sterisch gehinderte Amine allgemein als Lichtschutzmittel für organische Materialien, insbesondere für Polymere, bekannt. Es wurde auch bereits in der DE-A 2 126 954 vorgeschlagen, solche Polyalkylpiperidine als Mittel gegen das Ausbleichen von Farbphotographien zu verwenden. Es wurde weiterhin in der EP-A 0 011 051 vorgeschlagen, als Lichtschutzmittel für Farbphotographien bestimmte Polyalkylpiperidinderivate zu verwenden, die mindestens eine Phenolgruppe enthalten. Es handelt sich dabei um Polyalkylpiperidinester von Hydroxybenzylmalonsäuren.

In Weiterführung dieser Forschungsarbeiten wurde gefunden, dass Verbindungen, die sowohl einen sterisch gehinderten Hydrochinon- oder Resorcin-monoetherrest als auch einen Polyalkylpiperidinrest enthalten, eine überraschend verbesserte Wirkung besitzen.

Gegenstand der vorliegenden Erfindung ist daher ein farbphotographisches Aufzeichnungsmaterial, das in mindestens einer lichtempfindlichen Silberhalogenidemulsionsschicht, einer Zwischenschicht, einer Bildempfangsschicht und/oder einer Schutzschicht als Stabilisator mindestens eine Verbindung enthält, die in ihrem Molekül mindestens einen Hydrochinonmonoether- oder Resorcinmonoether-Rest, dessen freie OH-Gruppen sterisch gehindert sind, und mindestens einen Polyalkylpiperidinrest enthält.

Es handelt sich bei diesen Stabilisatoren insbesondere um Verbindungen der Formel I,

$[A]_a$—Z—$[B]_b$ (I)

worin a und b unabhängig voneinander eine ganze Zahl von 1 bis 5 sind und (a+b) eine Zahl von 2 bis 6 ist,

A ein einwertiger Hydrochinonmonoether- oder Resorcinmonoether-Rest ist, dessen freie OH-Gruppe sterisch gehindert ist,

Z ein (a+b)-wertiger organischer Rest ist, der die Gruppen A und B verbindet, und

B ein einwertiger Polyalkylpiperidinrest der Formel II oder III ist,

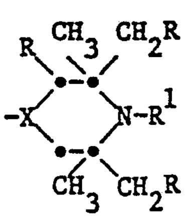

(II)

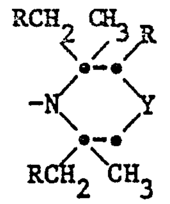

(III)

worin R Wasserstoff oder Methyl ist,

X eine Gruppe -CH oder einen 5- oder 6gliedrigen heterocyclischen Spiroring mit zwei O- oder N-Atomen bedeutet,

Y eine Gruppe $-CH_2-$ oder $-CH(R^2)-$ oder einen 5- oder 6gliedrigen heterocyclischen Spiroring mit zwei O- oder N-Atomen bedeutet,

$R^1$ Hydroxyl, $C_1$–$C_{12}$-Alkyl, $C_3$–$C_6$-Alkenyl, $C_3$–$C_4$-Alkinyl, $C_7$–$C_{12}$-Phenylalkyl, Glycidyl, durch Halogen, -CN, $-COOR^3$ oder $-CON(R^4)(R^5)$ substituiertes $C_1$–$C_4$-Alkyl, eine Gruppe $-CO-R^6$, $-CO-OR^3$, $-CO-N(R^4)(R^5)$, $-CH_2-CH(R^7)-OR^8$, $-SO-R^9$, $-SO_2-R^9$, $-OR^3$ oder $-OOC-R^6$ bedeutet, oder eine Gruppe $-Z^o-A$ bedeutet, worin $Z^o$ ein zweiwertiger organischer Rest ist und im Falle, dass b = 1 ist, $R^1$ auch eine Gruppe der Formel

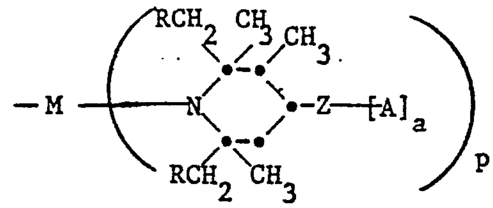

sein kann, worin p 1, 2 oder 3 ist und

M, wenn p = 1 ist, eine zweiwertige Gruppe ist und $C_2$–$C_{12}$-Alkylen, $C_4$–$C_8$-Alkenylen, Xylylen oder einen Rest der Formel $-CH_2-C{\equiv}C-CH_2-$

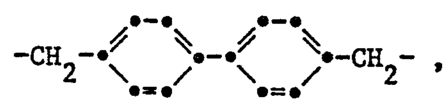

,

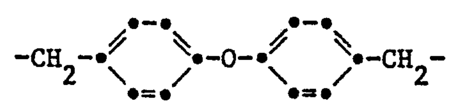

,

$-CH_2-COO-R^{10}-OOC-CH_2-$, $-CH_2-CH(OH)-CH_2-$, $-CH_2CH(OH)CH_2-D-CH_2CH(OH)CH_2-$, $-CH_2-CH(R^7)-OOC-R^{11}-COO-CH(R^7)-CH_2-$ oder $-CO-NH-G-NH-CO-$ darstellt, worin D einen zweiwertigen Rest der Formel $-O-R^{12}-O-$ oder $-OOC-R^{11}-COO-$ darstellt und G einen zweiwertigen aliphatischen, cycloaliphatischen, aromatischen oder aromatisch-aliphatischen Rest mit 6–15 C-Atomen bedeutet,

M, wenn p = 2 ist, einen dreiwertigen Rest der Formel

$T-[CH_2CH(OH)CH_2]_3$ oder

$R^{13}-[COO-CH(R^7)-CH_2]_3$

darstellt, worin T ein dreiwertiger Rest der Formel

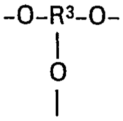 oder 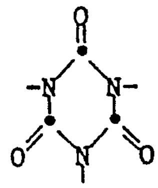 ist, und

M, wenn p = 3 ist, einen vierwertigen Rest der Formel

$Q-[CH_2CH(OH)CH_2-]_4$ oder

$R^{14}-[COO-CH(R^7)-CH_2-]_4$

darstellt, worin Q eine Gruppe der Formel

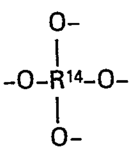 oder

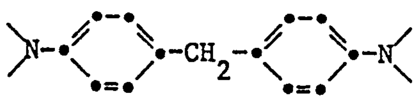 ist,

$R^2$ Hydroxyl, $-OR^{15}$, $-OOC-R^6$, $-OOC-N(R^4)(R^5)$, $-N(R^{16})-CO-R^6$ oder $-N(R^{16})-CO-N(R^4)(R^5)$ bedeutet, oder eine Gruppe $-Z^o-A$ bedeutet und im Falle, dass b = 1 ist, $R^2$ auch eine Gruppe der Formel

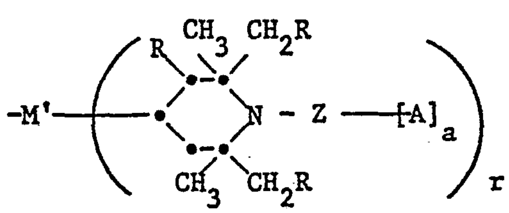

sein kann, worin r 1, 2 oder 3 ist und

M′, wenn r = 1 ist, eine zweiwertige Gruppe der Formel $-OOC-R^{11}-COO-$, $-OOC-NH-G-NH-COO-$, $-N(R^{16})-CO-R^{11}-CO-N(R^{16})-$ oder $-N(R^{17})-R^{18}-N(R^{17})-$, wenn r = 2 ist, eine dreiwertige Gruppe der Formel

$R^{19}[COO-]_3$ oder $R^{19}[CON(R^{16})-]_3$

und wenn r = 3 ist, eine vierwertige Gruppe der Formel

$R^{20}[COO-]_4$ oder $R^{20}[CON(R^{16})-]_3$

bedeutet,

$R^3$ $C_1$–$C_{12}$-Alkyl, Benzyl oder Cyclohexyl bedeutet,

$R^4$ $C_1$–$C_{12}$-Alkyl, Allyl, Cyclohexyl, Benzyl oder Phenyl und

$R^5$ Wasserstoff, $C_1$–$C_8$-Alkyl oder Allyl bedeuten und

$R^4$ und $R^5$ zusammen mit dem N-Atom einen 5- oder 6gliedrigen heterocyclischen Ring bilden,

$R^6$ Wasserstoff, $C_1$–$C_{12}$-Alkyl, $C_2$–$C_6$-Alkenyl, Chlormethyl, $C_5$–$C_{12}$-Cycloalkyl, $C_7$–$C_{12}$-Phenylalkyl, Phenyl, $C_7$–$C_{10}$-Alkylphenyl oder durch 1 oder 2 $C_1$–$C_4$-Alkylgruppen und eine Hydroxygruppe substituiertes Phenyl, Phenylmethyl oder Phenylethyl bedeutet,

$R^7$ Wasserstoff, $C_1$–$C_4$-Alkyl, $C_2$–$C_{13}$-Alkoxymethyl, Phenyl oder Phenoxymethyl bedeutet,

$R^8$ Wasserstoff, $C_1$–$C_{12}$-Alkyl, $-CO-R^6$ oder $-CO-N(R^4)(R^5)$ bedeutet,

$R^9$ $C_1$–$C_{12}$-Alkyl, Phenyl oder $C_7$–$C_{22}$-Alkylaryl bedeutet,

$R^{10}$ $C_2$–$C_{12}$-Alkylen, $C_4$–$C_8$-Oxaalkylen oder Cyclohexylen bedeutet,

$R^{11}$ eine direkte Bindung, $C_1$–$C_{12}$-Alkylen, $C_2$–$C_6$-Alkenylen, $C_6$–$C_{12}$-Cycloalkylen oder -Cycloalkenylen oder $C_6$–$C_{12}$-Arylen bedeutet,

$R^{12}$ $C_2$–$C_{12}$-Alkylen, $C_6$–$C_{12}$-Cycloalkylen, $C_6$–$C_{12}$-Arylen oder Phenylen-W-Phenylen- und W $-O-$, $-CH_2-$, $C(CH_3)_2$ oder $-SO_2-$ bedeutet,

$R^{13}$ ein dreiwertiger aliphatischer Kohlenwasserstoffrest mit 3–10 Kohlenstoffatomen oder ein dreiwertiger aromatischer Kohlenwasserstoffrest mit 6–10 C-Atomen ist,

$R^{14}$ einen vierwertigen aliphatischen Kohlenwasserstoffrest mit 4–10 C-Atomen oder einen vierwertigen aromatischen Kohlenwasserstoffrest mit 6–12 C-Atomen bedeutet,

$R^{15}$ Wasserstoff, $C_1$–$C_{12}$-Alkyl, Allyl oder Benzyl ist,

$R^{16}$ Wasserstoff, $C_1$–$C_{12}$-Alkyl, $C_5$–$C_{12}$-Cycloalkyl oder Benzyl ist,

$R^{17}$ Wasserstoff, $C_1$–$C_{12}$-Alkyl, Allyl, Benzyl, $C_2$–$C_{12}$-Alkanoyl oder Benzoyl bedeutet,

$R^{18}$ $C_2$–$C_{12}$-Alkylen, durch ein oder mehrere $-O-$ oder $-N(R^{16})-$ unterbrochenes $C_4$–$C_{16}$-Alkylen oder $C_6$–$C_{12}$-Cycloalkylen bedeutet,

$R^{19}$ ein dreiwertiger aliphatischer Rest mit 3–10 C-Atomen oder ein dreiwertiger aromatischer Rest mit 6–10 C-Atomen oder die Gruppe $N(CH_2)_3$ und

$R^{20}$ ein vierwertiger aliphatischer Rest mit 4–10 C-Atomen oder ein vierwertiger aromatischer Rest mit 6–12 C-Atomen ist.

Die Gruppe Z kann an den Rest A über dessen Ethergruppe oder direkt an den Benzolring von A gebunden sein. Die Bindung von Z an B erfolgt entweder an das N-Atom des Piperidinringes, wie es in Formel III ausgedrückt ist, oder in die 4-Stellung des Piperidinringes oder an einen in 4-Stellung befindlichen heterocyclischen Spiroring, wie es durch Formel II ausgedrückt ist. Z ist je nach dem Wert von a und b ein 2–6wertiger organischer Rest. Dieser kann neben Kohlenwasserstoff-Resten auch O, N, S oder P enthalten. Bevorzugt enthält er Ester- oder Amidgruppen, er kann aber auch heterocyclische Reste wie z.B.

einen s-Triazinrest oder einen Hydantoinrest enthalten. Die Auswahl von Z geschieht im allgemeinen nach seiner synthetischen Zugänglichkeit und nach seiner Eignung die Gruppen A und B dauerhaft zu verbinden. Der Einfluss von Z auf die stabilisierende Wirksamkeit der Verbindungen der Formel I ist im allgemeinen gering, während A und B die eigentlichen Wirkgruppen sind.

Die Polyalkylpiperidingruppen B sind dieselben, wie sie in Lichtstabilisatoren für Kunststoffe und Lacke bekannt sind. Je nach der Bindung an Z können die Gruppen B durch die Formel II oder III dargestellt werden. Wenn darin X oder Y heterocyclische Spiroringe sind, so kann es sich dabei z.B. um 2-Spiro-1,3-dioxolane, 2-Spiro-1,3-dioxane, 4-Spiromidazolin-2,5-dione, 2-Spirooxazolidin-4-one oder 5-Spirooxazolidin-4-one handeln. Beispielsweise kann eine Gruppe der Formel II eine solche der Formel

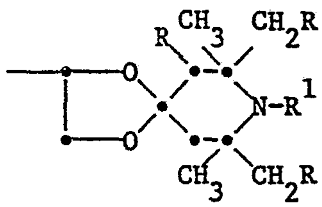

oder

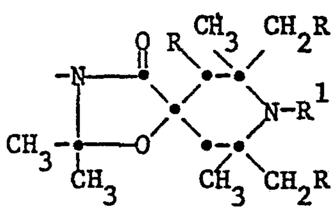

sein. Eine Gruppe der Formel III kann beispielsweise eine solche der Formel

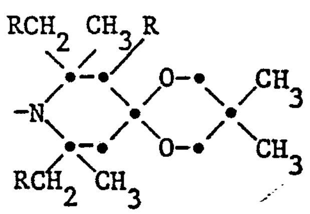

oder

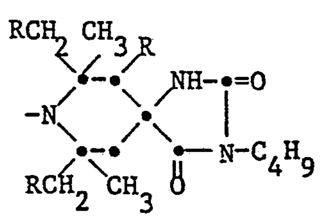

sein.

Die Gruppe $R^7$ als $C_1$–$C_4$-Alkyl kann z.B. Methyl, Ethyl, n-Propyl, Isopropyl, n-Butyl, Isobutyl oder tert. Butyl sein.

$R^5$ als $C_1$–$C_8$-Alkyl kann darüber hinaus auch z.B. Isoamyl, n-Hexyl, 2-Ethylbutyl, n-Octyl oder 2-Ethylhexyl sein. $R^1$, $R^3$, $R^4$, $R^6$,$R^8$, $R^9$, $R^{15}$, $R^{16}$ und $R^{17}$ als $C_1$–$C_{12}$-Alkyl können darüber hinaus auch z.B. n-Decyl, Isononyl oder n-Dodecyl sein. $R^7$ als $C_2$–$C_{15}$-Alkoxymethyl kann z.B. Methoxy-, Ethoxy-, Butoxy-, Hexyloxy-, Octyloxy- oder Dodecyloxymethyl sein.

$R^1$ als $C_3$–$C_6$-Alkenyl kann insbesondere Alkenylmethyl, wie z.B. Allyl, Methallyl, 2-Butenyl-1 oder 3,3-Dimethylallyl sein. $R^6$ als $C_2$–$C_6$-Alkenyl kann z.B. Vinyl, 2-Propenyl, Allyl, 2-Methylvinyl, 2,2-Dimethylvinyl oder Methallyl sein. $R^1$ als $C_3$–$C_4$-Alkinyl kann z.B. Propargyl oder 3-Methylpropargyl sein.

$R^1$ als durch Halogen, $-CN$, $-COOR^3$ oder $-CON(R^4)(R^5)$ substituiertes Alkyl kann z.B. Chlormethyl, 2-Chlorpropyl, Cyanomethyl, 2-Cyanoethyl, Ethoxycarbonylmethyl, Octyloxycarbonylmethyl, 2-Methoxy-, 2-Isopropoxy- oder 2-Hexyloxycarbonylethyl, N-Dimethylcarbamoylmethyl, N-Diallylcarbamoylethyl oder 2-(Morpholinocarbonyl)-ethyl sein.

$R^6$ und $R^{16}$ als $C_5$–$C_{12}$-Cycloalkyl können z.B. Cyclopentyl, Methylcyclopentyl, Cyclohexyl, Dimethylcyclohexyl, Cyclooctyl oder Cyclododecyl sein.

$R^1$ und R6 als $C_7$–$C_{12}$-Phenylalkyl können z.B. Benzyl, 2-Phenylethyl, 3-Phenylpropyl oder 3-Phenylbutyl sein. $R^6$ als $C_7$–$C_{10}$-Alkylphenyl kann z.B. Tolyl, Xylyl, Ethylphenyl, Isopropylphenyl oder tert. Butylphenyl sein. $R^9$ als $C_7$–$C_{22}$-Alkylaryl kann darüber hinaus auch z.B. Methylnaphthyl, Butylnaphthyl, Dibutylphenyl, Dioctylphenyl, Nonylphenyl oder Dodecylphenyl sein.

$R^{17}$ als $C_2$–$C_{12}$-Alkanoyl kann z.B. Acetyl, Propionyl, Isobutyryl, 2-Ethylbutyryl, n-Octanoyl, n-Decanoyl oder n-Dodecanoyl sein.

$R^4$ und $R^5$ können zusammen mit dem N-Atom, an das sie gebunden sind, einen 5- oder 6gliedrigen heterocyclischen Ring bilden. Dies kann z.B. ein Pyrrolidin-, Piperidin-, Morpholin- oder 4-Methylpiperazinring sein.

M, $R^{10}$, $R^{12}$ und $R^{18}$ als $C_2$–$C_{12}$-Alkylen können unverzweigtes Alkylen wie z.B. Di-, Tri-, Tetra-, Hexa-, Octa- oder Dodecamethylen oder verzweigtes Alkylen wie z.B. 1,2-Propylen, 1,2-Butylen, 2,2-Dimethyl-1,3-propylen oder 2,5,5-Trimethylhexamethylen sein. $R^{11}$ als $C_1$–$C_{12}$-Alkylen kann darüber hinaus auch Methylen darstellen.

$R^{10}$ als $C_4$–$C_8$-Oxaalkylen kann z.B. 3-Oxapentylen-1,5, 4-Oxaheptylen-2,6 oder 3,6-Dioxaoctylen-1,8 sein. $R^{18}$ als durch O oder $NR^{16}$ unterbrochenes $C_4$–$C_6$-Alkylen kann darüber hinaus auch z.B. 3-Azapentylen-1,5, 4-Azaheptylen-2,6 oder 3-(Methylaza)-pentylen-1,5 sein.

M als $C_4$–$C_8$-Alkenylen kann z.B. 2-Butenylen-1,4, 3-Hexenylen-1,6 oder 4-Octenylen-1,8 sein. $R^{11}$ als $C_2$–$C_6$-Alkenylen kann z.B. Vinylen, Methylvinylen oder 2-Butenylen-1,4 sein.

$R^{12}$ und $R^{18}$ als $C_6$–$C_{12}$-Cycloalkylen können z.B. 1,4-Cyclohexylen, 1,2-Cyclohexylen, 1,4-Endomethylencyclohexylen-1,2, 1,4-Dimethylencyclohexan, 4,4′-Dicyclohexylen oder 1,4-Decahydronaphthylen sein. $R^{11}$ und $R^{12}$ als $C_6$–$C_{12}$-Arylen können z.B. 1,2-Phenylen, 1,3-Phenylen, 1,4-Phenylen, 1,4-Naphthylen oder 4,4′-Diphenylen sein.

G als zweiwertiger aliphatischer, cycloaliphatischer, aromatischer oder aromatisch-aliphatischer Rest mit 6–15 C-Atomen kann z.B. Hexa-, Octa- oder Dodecamethylen, 2,2,4-Trimethylhexamethylen, Cyclohexylen, 4,4'-Dicyclohexylenmethan, 1,4-Phenylen, 2,4-Tolylen, 1,4-Naphthylen oder 4,4'-Diphenylenmethan sein.

$R^{13}$ und $R^{19}$ als dreiwertige aliphatische Reste mit 3–10 C-Atomen können z.B. Propan-1,2,3-triyl, 1,1,1-Trimethylenethan oder 1,1,1-Trimethylenpropan sein. $R^{13}$ und $R^{19}$ als dreiwertige aromatische Reste können z.B. Benzol-1,2,4-triyl oder Naphthalin-1,4,5-triyl sein.

$R^{14}$ und $R^{20}$ als vierwertige aliphatische Reste mit 4–10 C-Atomen können z.B. Butan-1,2,3,4-tetrayl oder Tetramethylenmethan sein. $R^{14}$ und $R^{20}$ als vierwertige aromatische Reste können z.B. Benzol-1,2,4,5-tetrayl, Naphthalin-1,4,5,8-tetrayl oder Diphenyl-3,4,3',4'-tetrayl sein.

Bevorzugt als Stabilisatoren sind Verbindungen der Formel I, worin A ein sterisch gehinderter Hydrochinonmonoether-Rest ist, sowie Verbindungen der Formel I, worin B ein Polyalkylpiperidinrest der Formel II oder III ist, in der R Wasserstoff ist. Letztere Verbindungen enthalten also einen 2,2,6,6-Tetramethylpiperidinrest.

Unter den Hydrochinonether-Derivaten sind drei Klassen von besonderer Bedeutung.

a) Verbindungen der Formel IV

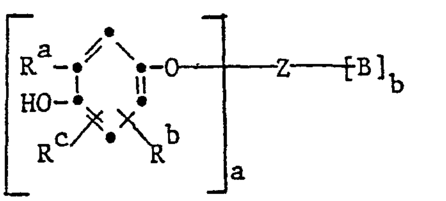

(IV)

worin a und b unabhängig voneinander eine Zahl von 1 bis 3 sind und (a+b) eine Zahl von 2 bis 4 ist,

$R^a$ ein einwertiger Kohlenwasserstoffrest ist, der die OH-Gruppe sterisch hindert,

$R^b$ Wasserstoff ist oder im Falle, dass a = 1 ist, auch ein Rest der Formel

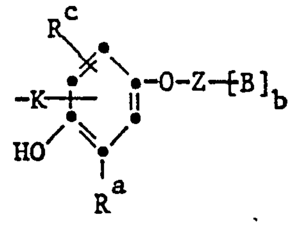

sein kann, worin K eine direkte Bindung oder eine zweiwertige Gruppe der Formel $-S-$, $-S-S-$, $-SO-$, $-SO_2-$, $-CH_2SCH_2-$, $-CH_2OCH_2-$, $-CH(R^{21})-$ oder $-N(R^{22})-$ ist, wobei $R^{21}$ Wasserstoff, $C_1$-$C_{12}$-Alkyl oder durch -S- oder -O- unterbrochenes $C_3$-$C_{15}$-Alkyl und $R^{22}$ Wasserstoff, $C_1$-$C_{18}$-Alkyl oder Phenyl bedeutet,

$R^c$ Wasserstoff oder $C_1$-$C_8$-Alkyl ist, und

Z und B die vorhin gegebene Bedeutung haben.

$R^{21}$ als $C_1$-$C_{12}$-Alkyl kann darin z.B. eine der für $R^1$ gegebenen Bedeutungen haben. $R^{22}$ als $C_1$-$C_{18}$-Alkyl kann darüber hinaus auch z.B. n-Tetradecyl, n-Hexadecyl oder n-Octadecyl sein. $R^{21}$ als durch -S- oder -O- unterbrochenes $C_3$-$C_{15}$-Alkyl kann z.B. Butylthiomethyl, 2-Dodecylthioethyl, 2-Methoxyethyl oder 2-Octyloxyethyl sein.

$R^a$ als sterisch hindernder Kohlenwasserstoffrest kann ein aliphatischer, cycloaliphatischer, aromatischer oder araliphatischer Rest sein. Insbesondere ist $R^a$ eine tertiäre Alkylgruppe mit 4–12 C-Atomen, wie z.B. tert.Butyl, tert.Amyl (1,1-Dimethylpropyl), 1,1,3,3-Tetramethylbutyl oder 1,1,3,3,5,5-Hexamethylhexyl, oder ist Cyclohexyl, Phenyl oder α,α-Dimethylbenzyl.

Z ist in diesen Verbindungen mit dem Hydrochinonrest durch eine Etherbindung verbunden. Bevorzugt ist Z ein aliphatischer Rest mit b Ester- oder Amidgruppen.

Unter den Stabilisatoren der Formel IV sind Verbindungen der Formel VII bevorzugt,

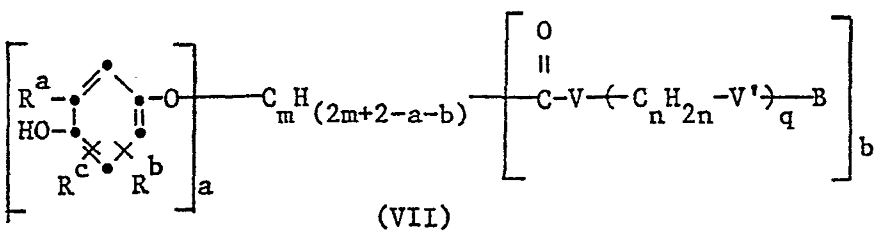

(VII)

worin m eine Zahl von 1 bis 20, n eine Zahl von 1 bis 4 und q null oder 1 ist, V -O- oder $-N(R^{22})-$ und V' eine direkte Bindung, -O- oder $-N(R^{22})-$ ist, a, b, $R^b$, $R^c$, $R^{22}$ und B die vorhin die für Formel IV gegebene Bedeutung haben und $R^a$ eine tertiäre Alkylgruppe mit 4-12 C-Atomen, Cyclohexyl, Phenyl oder α,α-Dimethylbenzyl ist, wobei im Falle, dass B ein Rest der Formel III ist, q 1 ist und V' eine direkte Bindung bedeutet.

Besonders bevorzugt sind Verbindungen der Formel VIIa und VIIb

(VIIa)

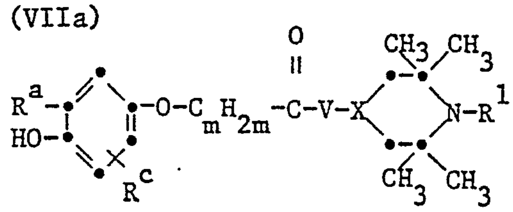

(VIIb)

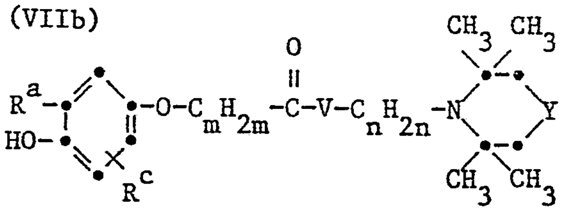

worin m eine Zahl von 1 bis 6 ist, n, V, $R^a$ und $R^c$ die für Formel VII angegebene Bedeutung haben und X, Y und $R^1$ die für die Formeln II und III angegebene Bedeutung haben.

b) Verbindungen der Formel V,

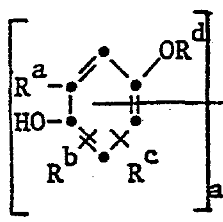
(V)

worin a, b, $R^a$, $R^b$, $R^c$, Z und B die für Formel IV gegebenen Bedeutungen haben und $R^d$ $C_1$–$C_{20}$-Alkyl, $C_3$–$C_{15}$-Alkoxyalkyl, $C_5$–$C_{12}$-Cycloalkyl, $C_7$–$C_{13}$-Aralkyl, Phenyl oder ein Rest $-C_mH_{2m}-CO-V-C_nH_{2n}-B$ ist, worin m, n und V die für Formel VII gegebene Bedeutung haben, oder $R^d$ zusammen mit einem orthoständigen $R^c$ und dem aromatischen Ring einen Cumaran- oder Chromanring bildet, und, wenn a = 1 ist, $R^d$ auch ein Rest der Formel

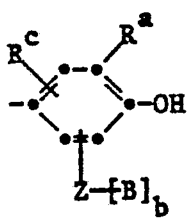

oder

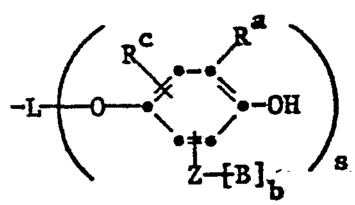

sein kann, worin s 1 oder 2 ist,

L, wenn s = 1 ist, $C_2$–$C_{12}$-Alkylen, das durch ein oder zwei O- oder S-Atome unterbrochen sein kann, $C_4$–$C_{10}$-Alkenylen, $C_4$–$C_6$-Alkinylen, $C_5$–$C_{12}$-Cycloalkylen, Xylylen oder einen Rest der Formel

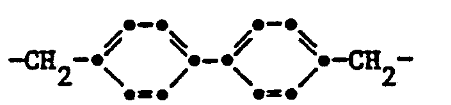
,

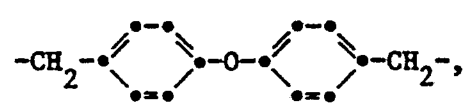
,

$-CH_2-CH(OH)-CH_2-$, $-CH_2CH(OH)CH_2-O-R^{12}-O-CH_2CH(OH)CH_2-$ oder $-CH_2COO-R^{10}-OOC-CH_2$ bedeutet, worin $R^{10}$ und $R^{12}$ die vorhin angegebene Bedeutung haben, und wenn s = 2 ist, L eine dreiwertige Gruppe der Formel

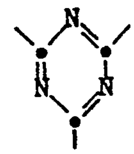

oder $HOCH_2-C(CH_2-)_3$

bedeutet.

$R^d$ ist ein einwertiger Rest und kann $C_1$–$C_{20}$-Alkyl sein, wie z.B. Methyl, Ethyl, Isopropyl, n-Butyl, Isoamyl, n-Hexyl, 2-Ethylbutyl, n-Octyl, 2-Ethylhexyl, n-Decyl, n-Dodecyl, n-Hexadecyl oder n-Octadecyl. $R^d$ kann $C_3$–$C_{15}$-Alkoxyalkyl sein wie z.B. 2-Methoxyethyl, 2-Butoxyethyl oder 2-Dodecyloxyethyl. $R^d$ als $C_5$–$C_{12}$-Cyclohexyl kann z.B. Cyclopentyl, Cyclohexyl, Methylcyclohexyl, Cyclooctyl oder Cyclododecyl sein. $R^d$ als $C_7$–$C_{13}$-Aralkyl kann z.B. Benzyl, 2-Phenylethyl, 1-Phenylisopropyl, 3-Phenylpropyl oder 2-Naphthylmethyl sein. Wenn $R^d$ zusammen mit einem orthoständigen $R^c$ und dem aromatischen Ring einen Cumaran- oder Chromanring bildet, so kann der Cumaran- oder Chromanring durch Alkylgruppen substituiert sein.

Wenn s = 1 ist, ist L eine zweiwertige Gruppe und kann eine $C_2$–$C_{12}$-Alkylengruppe sein, die durch O- oder S-Atome unterbrochen sein kann. Beispiele hierfür sind Di-, Tri-, Tetra-, Hexa-, Octa- oder Dodecamethylen, 1,2-Propylen, 1,2-Butylen, 2,2-Dimethyl-1,3-propylen, 2,5,5-Trimethylhexamethylen, 3-Oxapentylen-1,5, 4-Thiaheptamethylen oder 3,8-Dioxadecamethylen. L kann $C_4$–$C_{10}$-Alkenylen sein wie z.B. 2-Butenylen-1,4, 3-Hexenylen-1,6 oder 4-Octenylen-1,8 oder kann $C_4$–$C_6$-Alkinylen sein wie z.B. 2-Butinylen-1,4 oder 3-Hexinylen-1,6. L als $C_5$–$C_{12}$-Cycloalkylen kann z.B. 1,4-Cyclohexylen, 1,5-Cyclooctylen oder 4,4'-Dicyclohexylen sein.

$R^a$ ist vorzugsweise eine tertiäre Alkylgruppe mit 4–12 C-Atomen wie z.B. tert.Butyl, tert.Amyl, 1,1,3,3-Tetramethylbutyl oder 1,1,3,3,5,5-Hexamethylhexyl, oder ist Cyclohexyl, Phenyl oder α,α-Dimethylbenzyl.

Z ist in den Verbindungen der Formel V direkt an den aromatischen Ring gebunden und ist bevorzugt ein aliphatischer Rest, der b Ester- oder Amidgruppen enthält.

Unter den Stabilisatoren der Formel V sind Verbindungen der Formel VIIIa und VIIIb bevorzugt,

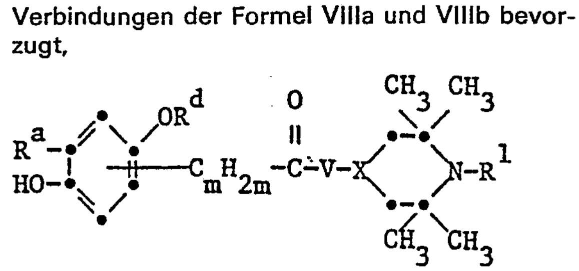

(VIIIa)

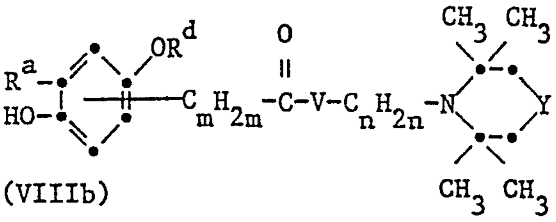

(VIIIb)

worin m eine Zahl von 1 bis 6 ist, n eine Zahl von 1 bis 4 ist, V -O- oder $-N(R^{22})-$ bedeutet, $R^a$ eine tertiäre Alkylgruppe mit 4–12 C-Atomen, Cyclohexyl, Phenyl oder α,α-Dimethylbenzyl ist, $R^d$ die vorhin gegebene Bedeutung hat, $R^{22}$ Wasserstoff, $C_1$–$C_{18}$-Alkyl oder Phenyl bedeutet und X, $R^1$ und Y die vorhin für Formel II und III gegebene Bedeutung haben.

c) Verbindungen der Formel VI,

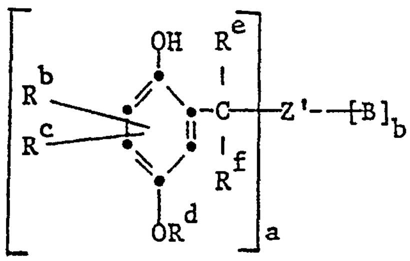

(VI)

worin a, b, $R^b$ und $R^c$ die für Formel IV gegebene Bedeutung haben und $R^b$ auch ein Rest der Formel IX sein kann,

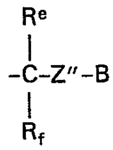

(IX)

$R^d$ die für Formel V gegebene Bedeutung hat, $R^e$ und $R^f$ $C_1$–$C_5$-Alkyl sind oder beide zusammen mit dem C-Atom, an das sie gebunden sind, einen Cycloalkan- oder Alkylcycloalkanring bilden oder eines von $R^e$ und $R^f$ zusammen mit dem C-Atom, an das sie gebunden sind, und mit dem Rest Z′ oder einem Teil des Restes Z′ einen Cycloalkan- oder Alkylcycloalkanring bilden, Z′ einen (a+b)-wertigen organischen Rest bedeutet, Z″ einen 2wertigen organischen Rest bedeutet und B die in Formel I gegebene Bedeutung hat.

$R^e$ und $R^f$ als $C_1$–$C_5$-Alkyl können z.B. Methyl, Ethyl, n-Propyl, n-Butyl oder Isobutyl sein. Bevorzugt sind $R^e$ und $R^f$ Methyl. Wenn $R^e$ und $R^f$ zusammen mit dem C-Atom, an das sie gebunden sind, einen Cycloalkan- oder Alkylcycloalkanring bilden, so kann dies z.B. ein Cyclopentan-, Cyclohexan-, Methylcyclohexan-, Dimethylcyclohexan- oder Cyclooctanring sein, bevorzugt ein Cyclohexanring.

Es kann auch nur einer der Reste $R^e$ und $R^f$ Teil eines Ringsystems sein, in diesem Falle zusammen mit dem Rest Z′ oder einem Teil des Restes Z′. Das Ringsystem ist cycloaliphatisch und ist insbesondere ein Cyclohexan- oder Alkylcyclohexanring.

In jedem Fall ist in den Verbindungen der Formel VI das C-Atom, an das $R^e$ und $R^f$ gebunden sind, ein tertiäres C-Atom und die dadurch gebildete tertiäre Gruppe bewirkt eine sterische Hinderung auf die phenolische OH-Gruppe.

Unter den Stabilisatoren der Formel VI sind solche bevorzugt, worin a 1 ist und Z′ ein aliphatischer Rest, der b Ester- oder Amidgruppen enthält, insbesondere Verbindungen der Formel X,

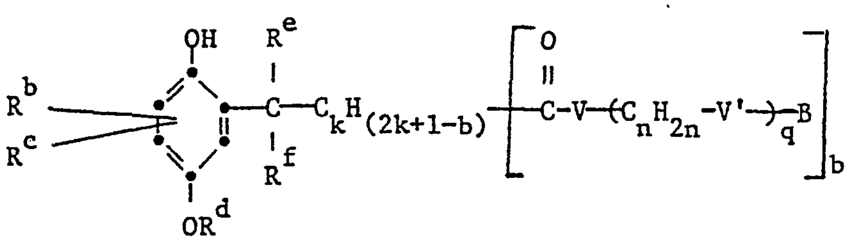

(X)

worin k null oder eine Zahl von 1 bis 20, n eine Zahl von 1 bis 4 und q null oder 1 ist, V -O- oder $-N(R^{22})-$ und V′ eine direkte Bindung, -O- oder $-N(R^{22})-$ bedeutet und b, B, $R^b$, $R^c$, $R^d$, $R^e$ und $R^f$ die vorhin für Formel VI gegebenen Bedeutungen haben, wobei im Falle, dass B ein Rest der Formel III ist, q 1 ist und V′ eine direkte Bindung bedeutet.

Besonders bevorzugt unter den Stabilisatoren der Formel VI sind Verbindungen der Formel XIa oder XIb,

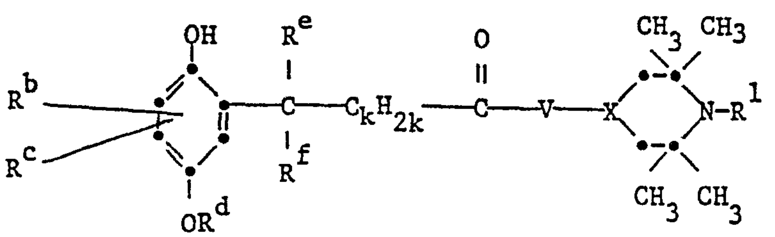

(XIa)

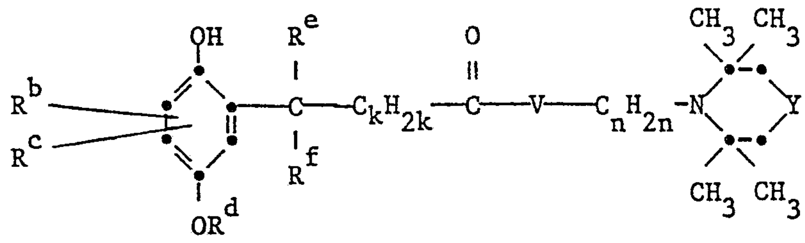

(XIb)

worin k null oder eine Zahl von 1 bis 6 ist, $R^b$ Wasserstoff oder eine Gruppe der Formel IXa oder IXb ist,

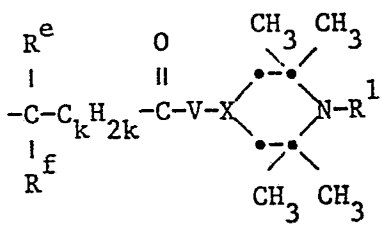

(IXa)

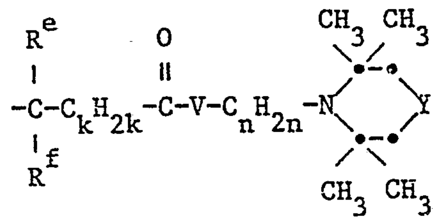

(IXb)

$R^e$ und $R^f$ Alkyl sind
n, $R^c$, $R^d$ und V die vorhin gegebene Bedeutung haben und
X, $R^1$ und Y die für die Formeln II und III gegebene Bedeutung haben.

Beispiele für Verbindungen der Formel IV sind die Verbindungen der folgenden Formeln:

1. 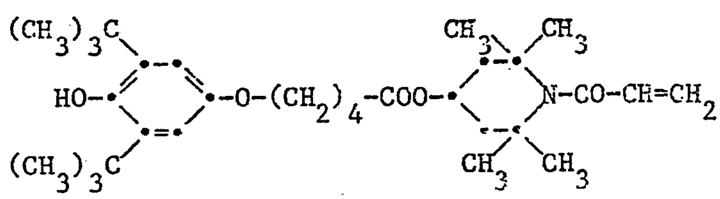

2. 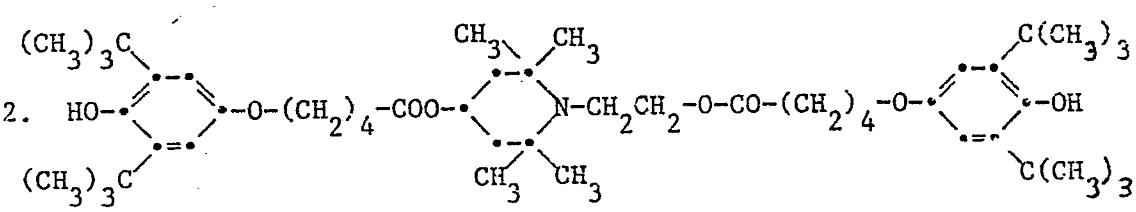

3. 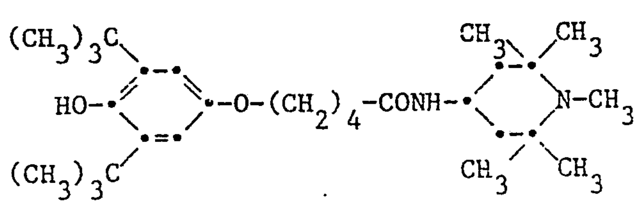

4. 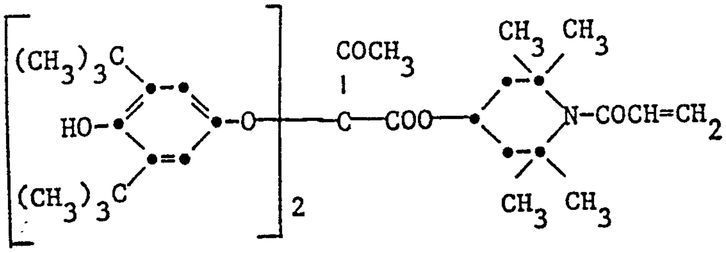

5. 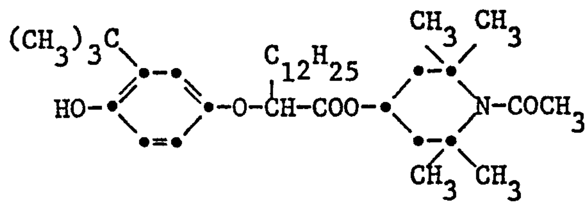

6. 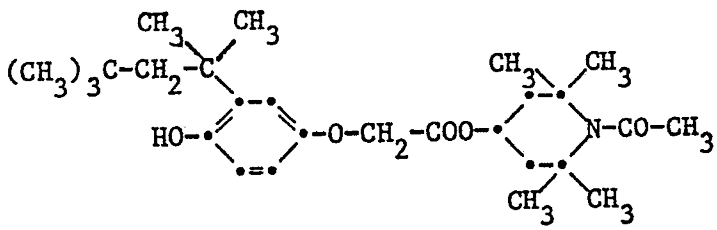

7. 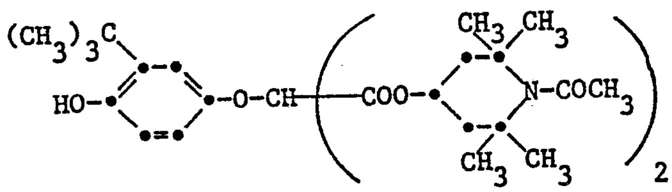

8. 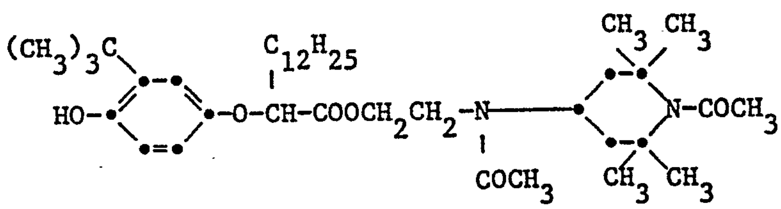

9. 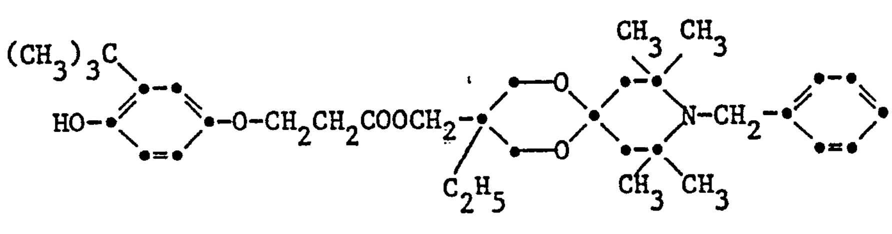

10. 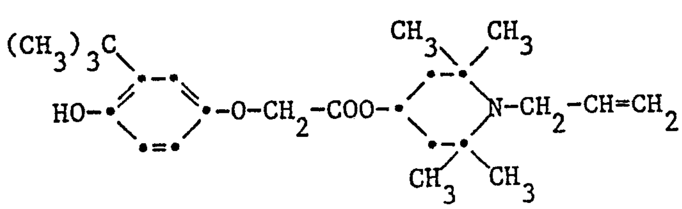

11. 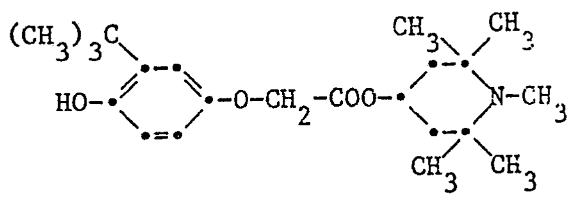

12. 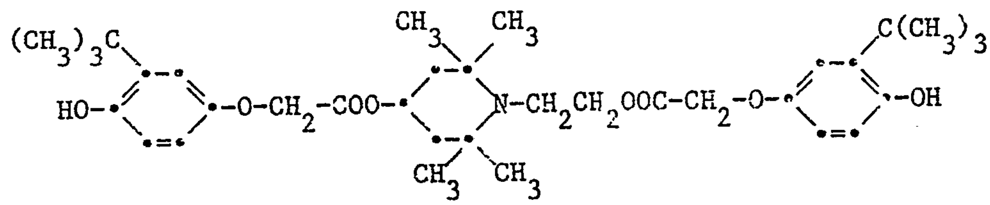

13. 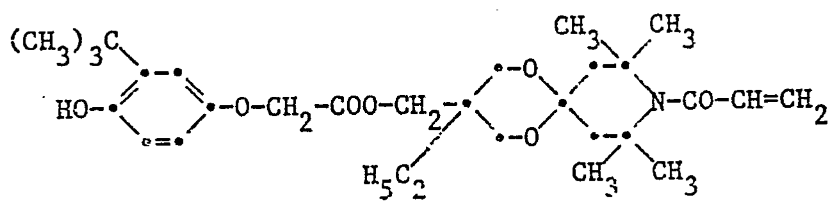

14. 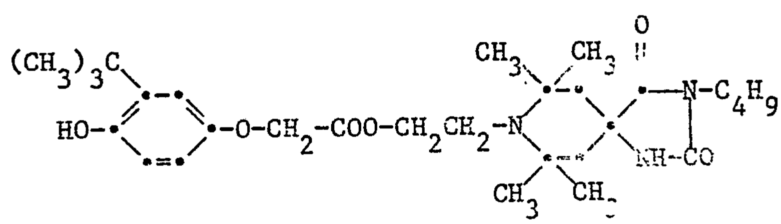

15. 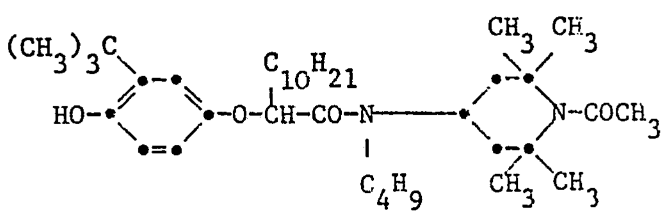

16. 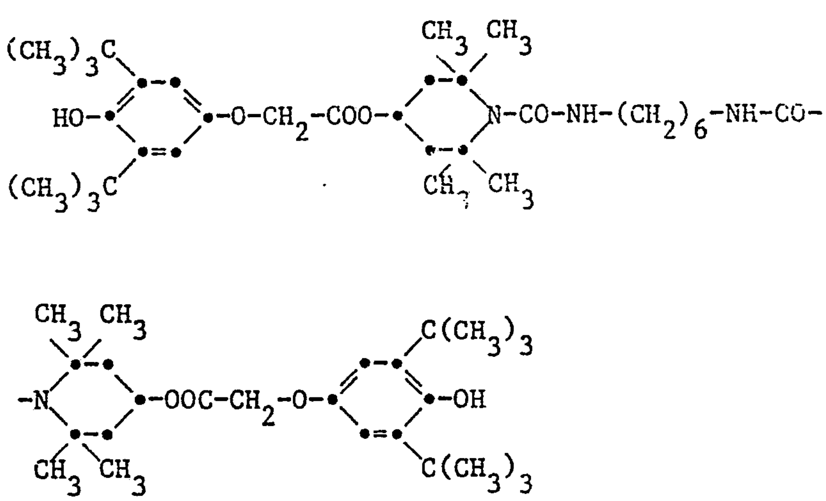

17. 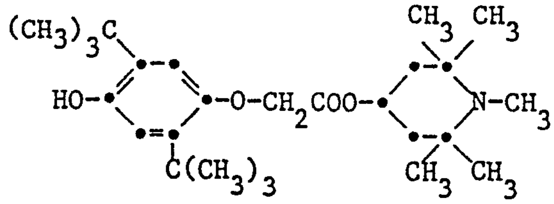

18. 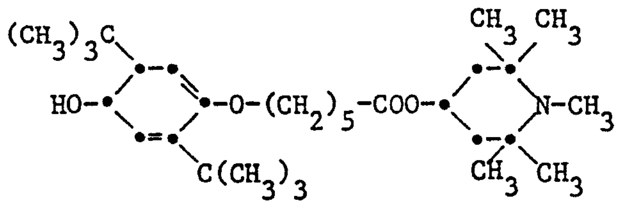

19. 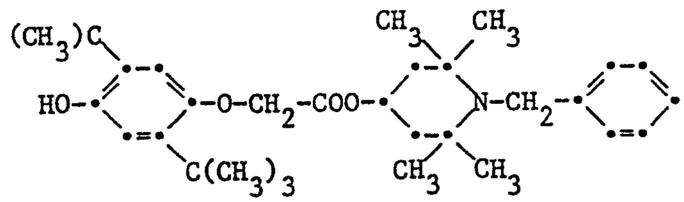

Beispiele für Verbindungen der Formel V sind die Verbindungen der folgenden Formeln:

20. 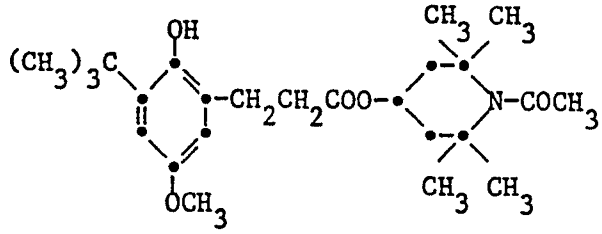

21. 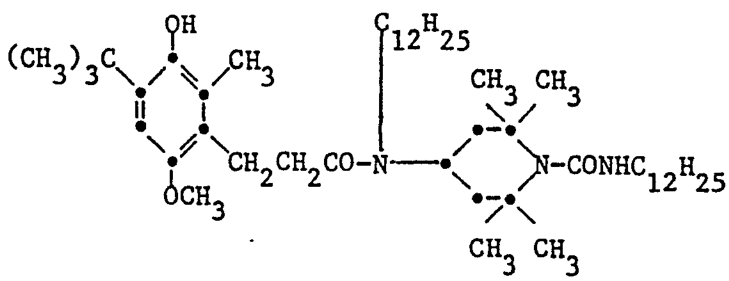

22. 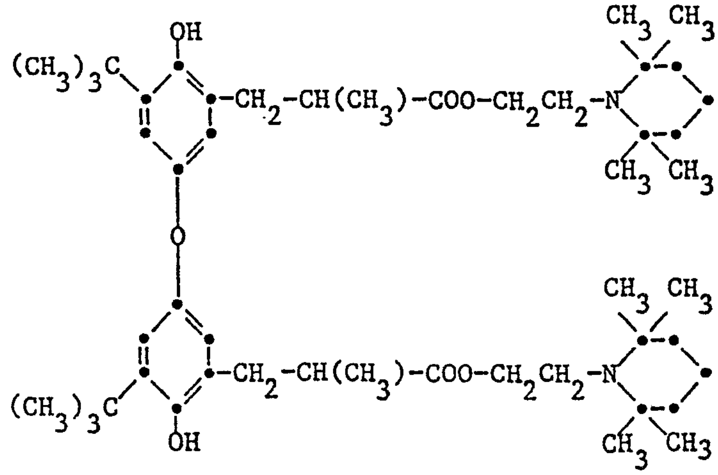

Beispiele für Verbindungen der Formel VI sind die Verbindungen der folgenden Formeln:

23. 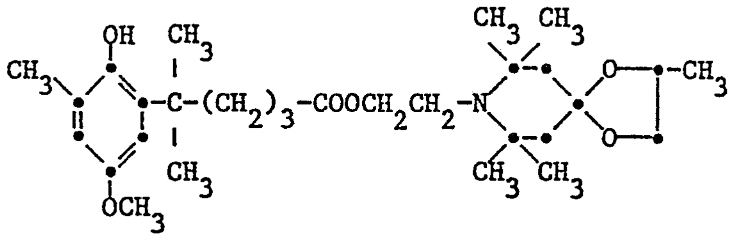

24. 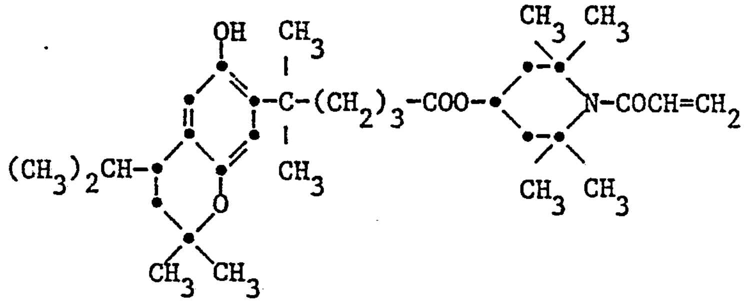

25. 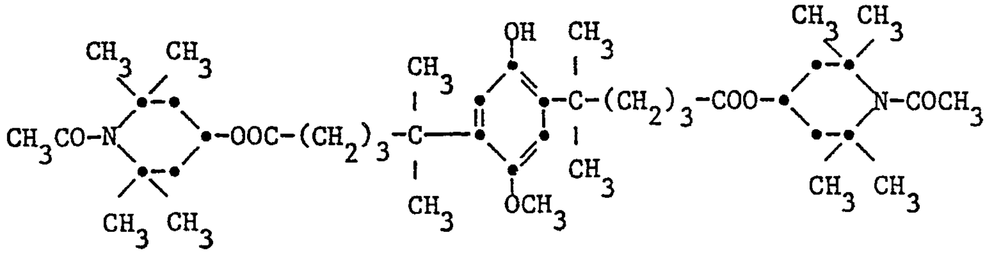

26. 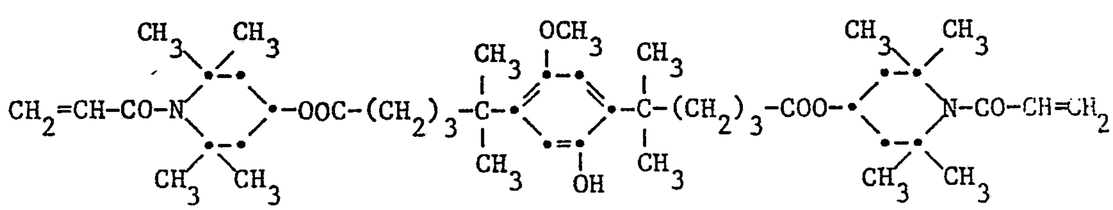

27. 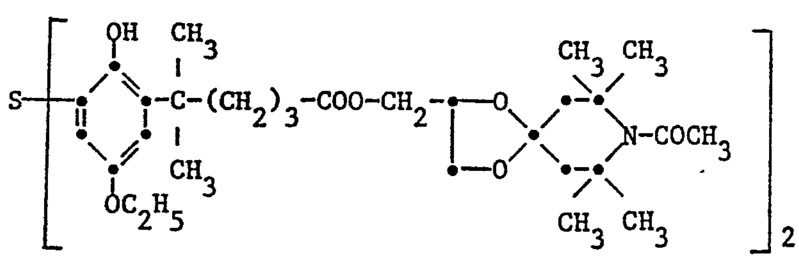

28. 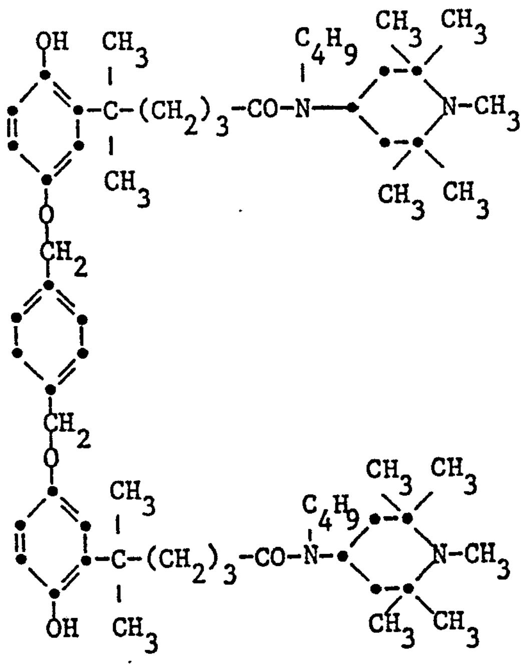

29. 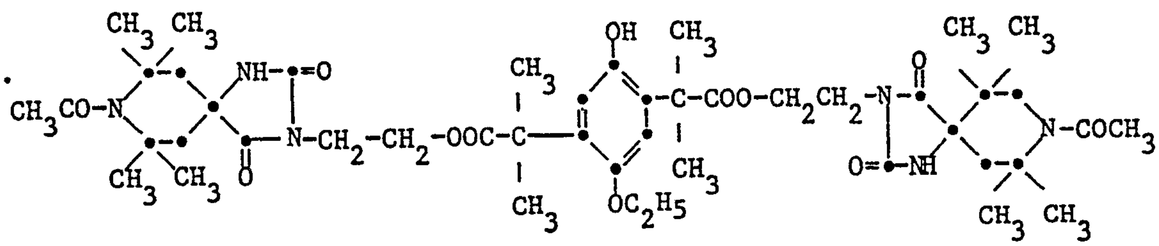

30. 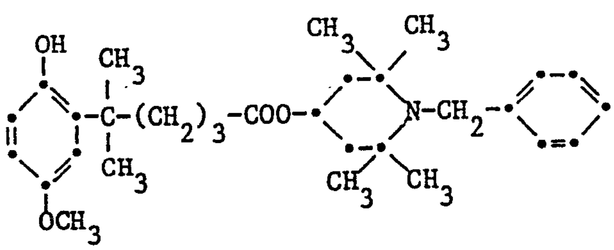

31. 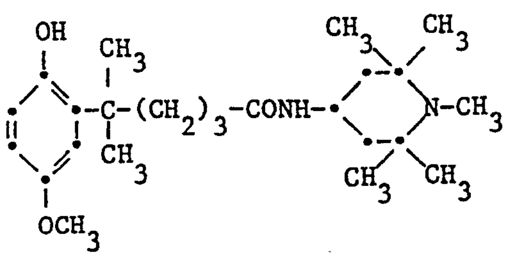

32. 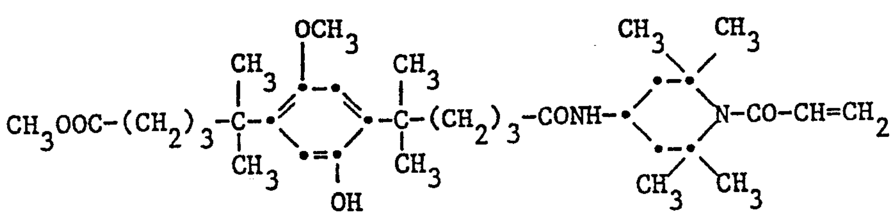

33. 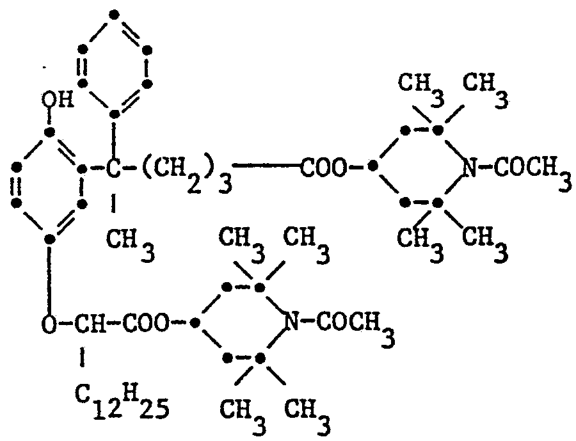

34. 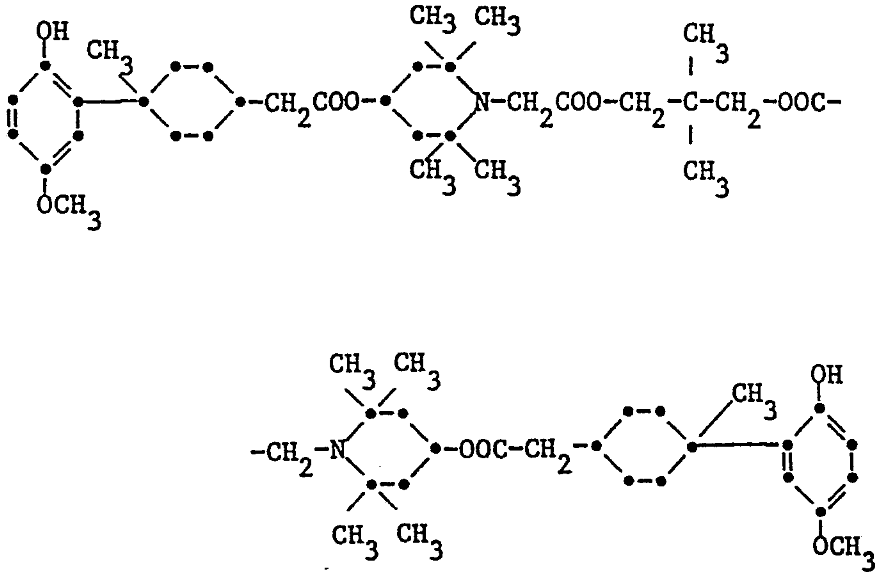

35. 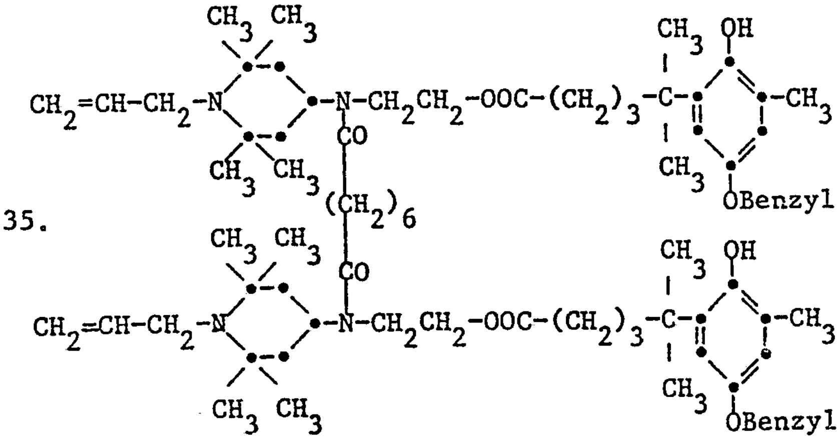

36. 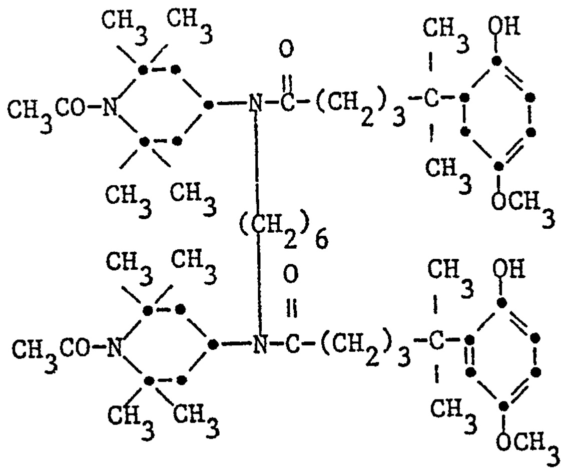

37. 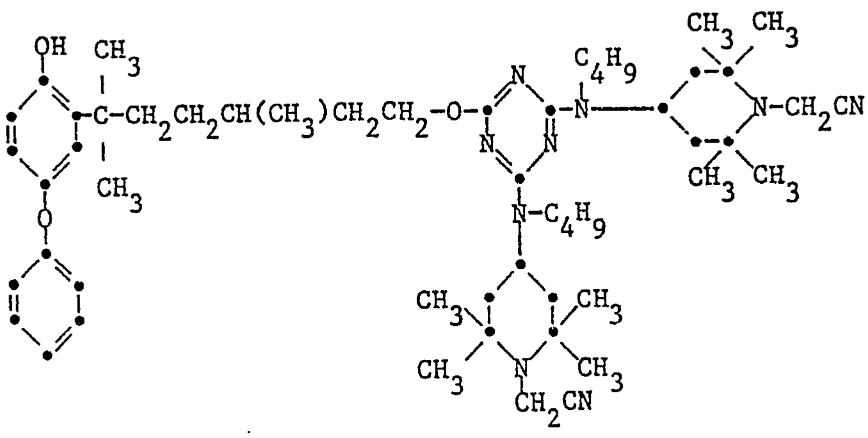

38. 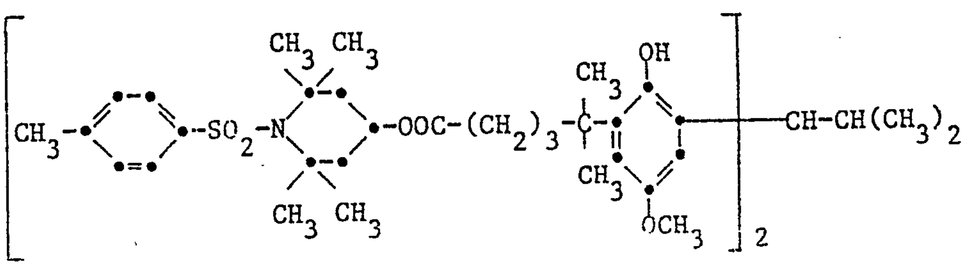

39. 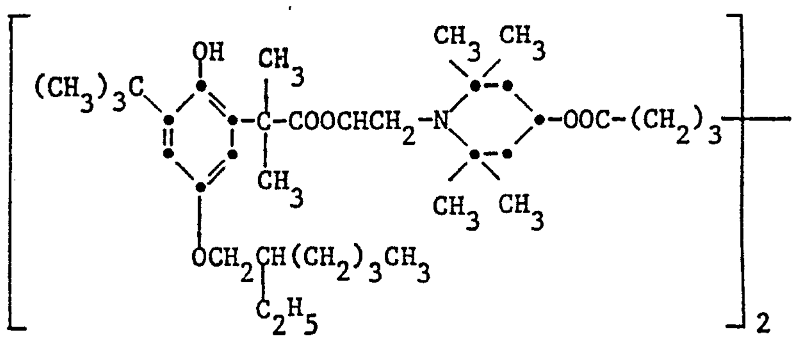

40. 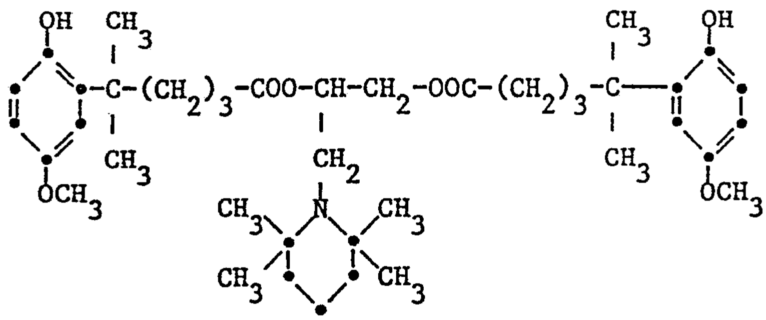

41. 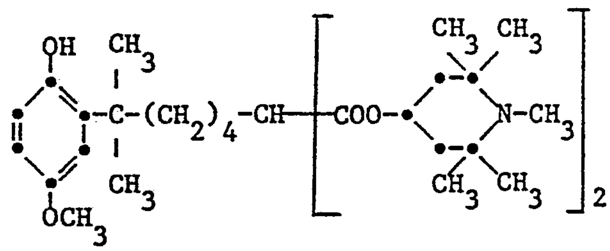

Die Verbindungen der Formel I sind neue Verbindungen und als solche ebenfalls Gegenstand der Erfindung. Ihre Herstellung kann nach verschiedenen Methoden geschehen, deren Prinzip meist eine Verknüpfung der Reste A mit den Resten B über das Bindeglied Z ist.

So können z.B. Verbindungen der Formel IV durch Veretherung von a Molen eines Hydrochinones der Formel XII mit einer Halogenverbindung XIII (Hal = Halogen)

a 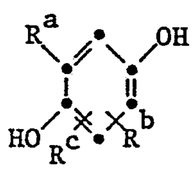 + $(Hal)_aZ[B]_b$ ⟶ IV

XII XIII

in Gegenwart von a Molen einer Base nach den üblichen Methoden der Veretherung von Phenolen hergestellt werden.

In alternativer Weise kann man jedoch auch das Hydrochinon zuerst mit einer Halogenverbindung verethern, die eine zusätzliche reaktive Gruppe hat, und bindet in einem zweiten Reaktionsschritt den Rest B über diese Gruppe an Z. Diese Gruppe kann insbesondere eine Ester- oder Amidgruppe sein. Für die Herstellung von Verbindungen der Formel VII kann dieses zweistufige Verfahren in folgender Weise formelmässig beschrieben werden:

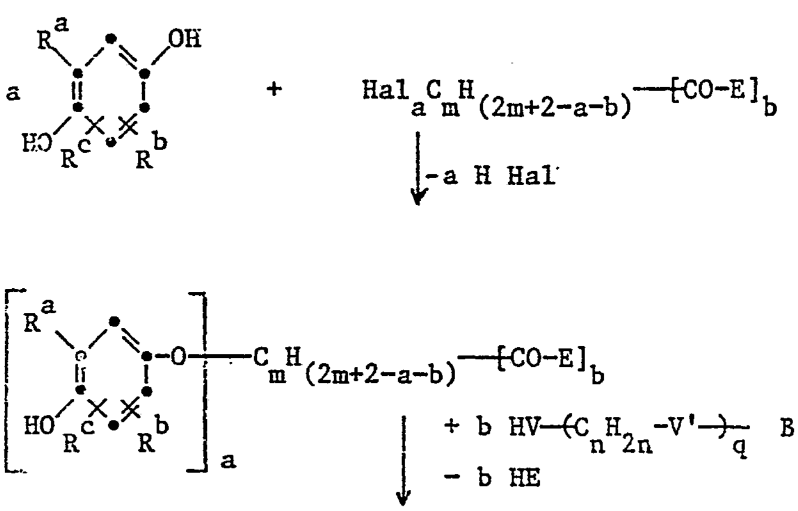

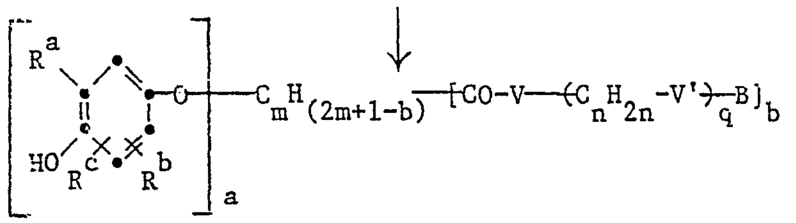

(VII)

Darin bedeutet E Hydroxyl oder Halogen, vorzugsweise jedoch Alkoxy mit 1-4 C-Atomen.

In ähnlicher Weise können Verbindungen der Formel V, in denen Z eine Ester- oder Amidgruppe enthält, durch Veresterung oder Amidierung einer Hydrochinon-monoether-carbonsäure mit einem HO- oder $R^{22}$ NH-Derivat eines Polyalkylpiperidins hergestellt werden.

Beispielsweise verläuft die Herstellung von Verbindungen der Formel VIIIa nach folgendem Schema:

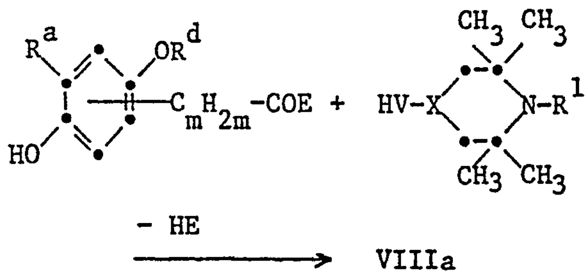


VIIIa

Eine Variante ist die Durchführung dieser Reaktion mit einer unveretherten Hydrochinonverbindung ($R^d$ = H) und anschliessende Einführung des Restes $R^d$ durch partielle Veretherung. Diese Variante empfiehlt sich vor allem dann, wenn $R^d$ ein zwei- oder dreiwertiger Rest ist.

In analoger Weise lassen sich Verbindungen der Formel VI, in denen Z′ Ester- oder Amidgruppen enthält, herstellen. Im Falle der Herstellung von Verbindungen der Formel X verläuft die Reaktion nach folgendem Schema:

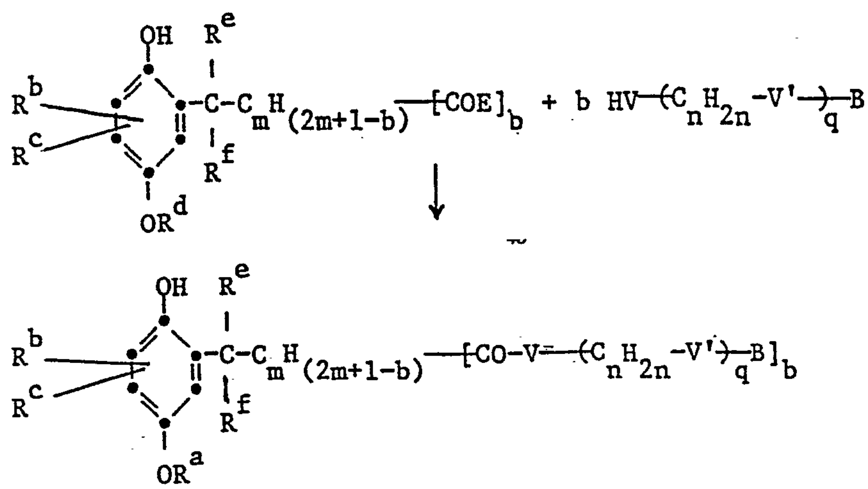

(X)

Auch hier kann wahlweise die Einführung von $R^d$ vor oder nach der Einführung von B geschehen.

In allen Verbindungen der Formel I kann die Einführung des Restes $R^1$ in alternativer Weise auch als letzter Schritt der Synthese geschehen. Sie geschieht nach den üblichen Methoden der N-Substitution sterisch behinderter Piperidine, die in vielen Patentschriften ausführlich beschrieben sind.

Insgesamt ist die Synthese von Verbindungen der Formel I stets eine mehrstufige Operation, und die Reihenfolge der einzelnen Schritte kann in jedem einzelnen Fall entsprechend variiert werden.

Die erfindungsgemässen Stabilisatoren können allein oder zusammen mit anderen Verbindungen in bekannter Weise in ein photographisches Material eingearbeitet werden.

In der Regel werden die Stabilisatoren allein oder zusammen mit anderen Verbindungen, insbesondere mit den Farbkupplern, in Form einer Dispersion in das photographische Material eingearbeitet, wobei diese Dispersion entweder kein Lösungsmittel oder hoch- oder tiefsiedende Lösungsmittel oder ein Gemisch solcher Lösungsmittel enthält. Eine weitere geeignete Einarbeitungsform besteht darin, dass man die Stabilisatoren allein oder zusammen mit weiteren Verbindungen zusammen mit einem Polymer in Form eines Latex in das photographische Material einarbeitet.

Die Dispersionen werden dann zur Herstellung der Schichten von farbphotographischen Aufzeichnungsmaterialien verwendet. Diese Schichten können z.B. Zwischen- oder Schutzschichten, insbesondere jedoch lichtempfindliche (blau-, grün- und rot-empfindliche) Silberhalogenidemulsionsschichten sein, in denen bei der Entwicklung des belichteten Aufzeichnungsmaterials aus den entsprechenden Farbkupplern die Blaugrün (Cyan)-, Purpur (Magenta)- und Gelbfarbstoffe gebildet werden.

Die Silberhalogenidschichten können beliebige Farbkuppler, insbesondere Blaugrün-, Purpur- und Gelb-Kuppler, die zur Bildung der genannten Farbstoffe und damit der Farbbilder verwendet werden, enthalten.

Da das Substrat die Wirkung und Stabilität der Stabilisatoren beeinflusst, werden Substrate (Lösungsmittel, Polymere) bevorzugt, die zusammen mit den Stabilisatoren eine möglichst gute Beständigkeit der zu stabilisierenden Materialien ergeben.

In der Regel werden die Stabilisatoren in Schichten eingearbeitet, die zusätzlich eine nach üblichen Methoden hergestellte und sensibilisierte Silberhalogenid-Dispersion enthalten. Sie können jedoch auch in zu Silberhalogenid enthaltenden Schichten benachbarten Schichten vorhanden sein.

Die erfindungsgemässen photographischen Materialien besitzen einen üblichen Aufbau und Komponenten, die die Wirksamkeit der Stabilisatoren verstärken oder zumindest nicht nachteilig beeinflussen.

Im photographischen Aufzeichnungsmaterial gemäss vorliegender Erfindung können die Stabilisatoren ausser mit den Farbkupplern zusätzlich auch mit Ultraviolettabsorbern oder anderen Lichtschutzmitteln in der gleichen Schicht kombiniert werden. Wenn die Diffusionstransfermethode angewendet wird, kann der Stabilisator auch in eine Empfangsschicht eingearbeitet werden.

Die erfindungsgemässen farbphotographischen Materialien können in bekannter Weise verarbeitet werden. Ferner können sie im Verlauf oder nach der Verarbeitung in einer Weise behandelt werden, die ihre Stabilität weiter erhöht, beispielsweise durch die Behandlung in einem Stabilisatorbad oder durch das Aufbringen eines Schutzüberzuges.

Die erfindungsgemäss einzusetzenden Stabilisatoren eignen sich in gewissen Fällen auch zum Schutz farbphotographischer Schichten, in denen die Farbstoffe direkt in die Emulsion eingelagert werden und das Bild durch selektive Bleichung erzeugt wird.

Die Menge des Stabilisators oder der Stabilisatoren kann in weiten Grenzen schwanken und liegt etwa im Bereich von 1 bis 2000 mg, vorzugsweise 100 bis 800 und insbesondere 200–500 mg pro $m^2$ der Schicht, in die es (sie) eingearbeitet wird (werden).

Falls das photographische Material einen oder mehrere UV-Absorber enthält, so kann dieser mit dem Stabilisator zusammen in einer Schicht oder auch in einer benachbarten Schicht vorhanden sein. Die Menge an UV-Absorber kann in weiten Grenzen schwanken und liegt etwa im Bereich von 200–2000 mg, vorzugsweise 400–1000 mg pro $m^2$ der Schicht. Beispiele für geeignete UV-Absorber sind solche vom Benzophenon-, Acrylnitril-, Thiazolidon-, Benztriazol-, Oxazol-, Thiazol- und Imidazoltyp.

Die mit dem erfindungsgemässen Aufzeichnungsmaterial durch Belichtung und Entwicklung erhaltenen Farbbilder zeigen eine sehr gute Lichtechtheit gegenüber sichtbarem und ultraviolettem Licht. Die erfindungsgemässen Stabilisatoren sind praktisch farblos, so dass es zu keiner Verfärbung der Bilder kommt; ausserdem sind sie gut verträglich mit den üblichen, in den einzelnen Schichten vorhandenen photographischen Zusatzstoffen. Aufgrund ihrer guten Wirksamkeit kann man ihre Einsatzmenge herabsetzen und vermeidet so ihre Ausfällung oder ihr Auskristallisieren, wenn man sie als organische Lösung in die wässerigen Bindemittelemulsionen, die für die Herstellung photographischer Schichten verwendet werden, einarbeitet. Die einzelnen nach der Belichtung des photographischen Aufzeichnungsmaterials notwendigen Verarbeitungsschritte zur Herstellung der Farbbilder werden durch die Stabilisatoren nicht nachteilig beeinflusst. Ferner kann die bei blauempfindlichen Emulsionen häufig auftretende sogenannte Druckschleierbildung weitgehend zurückgedrängt werden. Diese kann z.B. auftreten, wenn auf photographische Materialien (Silberhalogenidemulsionsschichten, die sich auf einem Träger aus natürlichen oder synthetischen Materialien befinden) mechanische Beanspruchungen, z.B. Drehen, Biegen oder Reiben, während der Herstellung oder während der Behandlung vor der Entwicklung ausgeübt werden. (T.H. James, The Theory of Photographic Process, 4. Auflage, Macmillan, New York, N.Y. 1977, Seite 23 ff., S. 166 ff.)

Die nachfolgenden Beispiele erläutern die Herstellung der erfindungsgemässen Verbindungen und ihre Verwendung in einem farbphotographischen Aufzeichnungsmaterial. Die Temperaturen sind darin als °C angegeben.

Beispiel 1

33,2 g tert.-Butylhydrochinon, 41,35 g Bromessigsäure-methylester und 37,3 g Kaliumcarbonat (wasserfrei) werden in 300 ml Aceton unter Stickstoff während 3 Tagen am Rückfluss erhitzt. Das Lösungsmittel wird unter vermindertem Drück entfernt, und 500 ml Äther zugegeben. Diese Ätherlösung wird mit Wasser gewaschen, mit Magnesiumsulfat getrocknet und eingedampft. Der Rückstand wird unter Vakuum destilliert. Das braune Destillat, das bei 138–140° und $10^{-3}$ mmHg erhalten wurde, wird mittels Säulenchromatographie gereinigt. Der so erhaltene 2-(3-tert.-Butyl - 4 - hydroxy - phenoxy) - essisäure - methylester kann aus Hexan kristallisiert werden. Smp. 87–89°.

2,38 g dieser Verbindung, 2,0 g 1-Allyl-4-hydroxy-2,2,6,6-tetramethylpiperidin und 0,1 g Dibutylzinnoxid werden in 80 ml Xylol auf Rückfluss erhitzt. Während drei Stunden wird ein azeotropes Methanol/Xylol-Gemisch langsam abdestilliert. Das restliche Lösungsmittel wird unter vermindertem Drück eingedampft und der Rückstand durch Säulenchromatographie gereinigt. So erhält man 2-(3-tert.-Butyl-4-hydroxy-phenoxy)-essigsäure - (1-allyl-2, 2, 6, 6-tetramethylpiperidin-4-yl)-ester als leicht braunes Öl (Verbindung Nr. 10).

Verwendet man anstelle von 1-Ally-4-hydroxy-2,2,6,6-tetramethylpiperidin eine entsprechende Menge 1-Methyl-4-hydroxy-2,2,6,6-tetramethylpiperidin und verfährt im übrigen wie oben geschrieben, so erhält man 2-(3-tert.-Butyl-4-hydroxy-phenoxy)-essigsäure-(1-methyl-2,2,6,6-tetramethylpiperidin-4-yl)-ester als leicht braunes Öl (Verbindung Nr. 11).

In analoger Weise erhält man bei Umsetzung von 3-tert.-Butyl-4-hydroxy-phenoxy-essigsäuremethylester mit 1-(2-Hydroxyethyl)-4-hydroxy-2,2,6,6-tetramethylpiperidin im Molverhältnis 2:1 das 1-[2-(3-tert.-Butyl-4-hydroxyphenoxyacetoxy)-ethyl]-4-(3-tert.-butyl-4-hyxdroxyphenoxyacetoxy)-2,2,6,6-tetramethylpiperidin als weisses Kristallisat, das bei 74–77° schmilzt (Verbindung Nr. 12).

Analog erhält man aus dem obengenannten Methylester und 3-Butyl-7,7,9,9-tetramethyl-8-(2-hydroxyethyl)-2,4-dioxo-1,3,8-triaza-spiro-[4.5]decan im Molverhältnis 1:1 das 8-[2-(3-tert.-Butyl-4-hydroxyphenoxyacetoxy)-ethyl]-3-butyl-7,7,9,9-tetramethyl-2,4-dioxo-1,3,8-triaza-spiro[4.5]decan, das bei 177–179° schmilzt (Verbindung Nr. 14).

Analog erhält man aus dem obengenannten Methylester und 3-Ethyl-3-hydroxymethyl-8,8,10,10-tetramethyl-1,5-dioxa-9-aza-spiro[5.5]undecan im Molverhältnis 1:1 das 3-(3-tert.-Butyl-4-hydroxyphenoxyacetoxymethyl)-3-ethyl-8,8,10,10-tetramethyl-1,5-dioxa-9-aza-spiro[5.5]undecan als hellbraunes Öl. 3,75 g dieser Verbindung werden in 60 ml Ethylacetat gelöst. Nach der Zugabe von 0,9 g Trimethylamin wird die Lösung auf -5° gekühlt und eine Lösung von 0,75 g Acrylsäurechlorid in 10 ml Ethylacetat tropfenweise zugegeben. Das Gemisch wird 3 h bei -5° gerührt und dann auf Raumtemperatur erwärmt. Die Lösung wird mit Wasser gewaschen, getrocknet und eingedampft. Der Rückstand wird durch Säulenchromatographie gereinigt. Man erhält so die N-Acryloyl-Verbindung (Verbindung Nr. 13) als weisse Kristalle, die bei 163–165° schmelzen.

Beispiel 2

Aus 2-Bromtetradecansäure-ethylester und tert.-Butylhydrochinon erhält man bei analoger Arbeitsweise wie in Beispiel 1 beschrieben den 2-(3-tert.-Butyl-4-hydroxyphenoxy)- tetradecansäure-ethylester als weisses Kristallisat, das bei 51–53° schmilzt.

21 g dieser Verbindung, 10 g 1-Acetyl-4-hydroxy-2,2,6,6-tetramethylpiperidin und 0,5 g Dibutylzinnoxid werden in 250 ml Xylol auf Rückfluss erhitzt. Das entstehende Ethanol wird im Azeotrop mit Xylol während 24 h abdestilliert. Die resultierende Lösung wird im Vakuum eingedampft und der Rückstand durch Säulenchromatographie gereinigt. Man erhält so den 1-Acetyl-2,2,6,6-tetramethyl-4-piperidinylester der 2-(3-tert.-Butyl-4-hydroxyphenoxy)-tetradecansäure (Verbindung Nr. 5) als weisses Kristallisat, das bei 106–108° schmilzt.

Beispiel 3

8 g 2,5-Bis-(5-methoxycarbonyl-2-methylpentyl-2)-4-methoxyphenol, 6,3 g 4-Hydroxy-2,2,6,6-tetramethylpiperidin und 0,2 g Dibutylzinnoxid werden in 150 ml Xylol zum Rückfluss erhitzt. Das entstehende Methanol wird im Azeotrop mit Xylol während 6 h abdestilliert. Das restliche Xylol wird im Vakuum abdestilliert, der Rückstand wird aus Acetonitril umkristallisiert, und man erhält das 2,5-Bis[5-(2,2,6,6-tetramethylpiperidin-4-yloxycarbonyl)-2-methylpent-2-yl]-4-methoxyphenol als weisses Kristallisat vom Smp. 165–168°.

5,3 g dieser Verbindung werden in 50 ml Ethylacetat gelöst. Nach Zugabe von 2 g Triethylamin wird die Lösung auf -5° gekühlt und eine Lösung von 1,5 g Acrylsäurechlorid in 10 ml Ethylacetat zugetropft. Das Gemisch wird eine Stunde bei -5° und eine weitere Stunde bei 0° gerührt. Die resultierende Lösung wird mit 1n Salzsäure und mit Wasser gewaschen, getrocknet und eingedampft. Der Rückstand wird durch Säulenchromatographie gereinigt. Man erhält 2,5-Bis[5-(1-acryloyl-2,2,6,6-tetramethylpiperidin-4-yloxycarbonyl)-2-methylpent-2-yl]-4-methoxyphenol als weisses Kristallisat, das bei 50–52° schmilzt (Verbindung Nr. 26).

Beispiel 4

0,087 g des Gelbkupplers der Formel

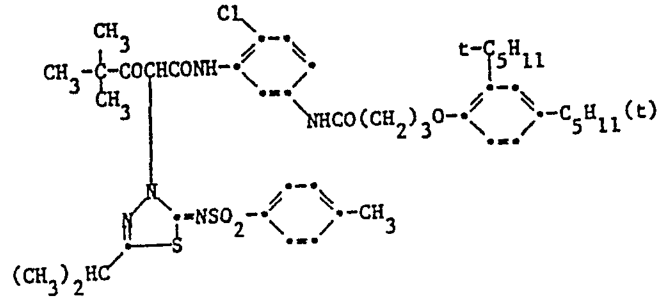

und 0,026 g eines der in der nachfolgenden Tabelle angegebenen Stabilisatoren werden in 2,0 ml eines Gemisches von Trikresylphosphat/Ethylacetat (1,5 g in 100 ml) gelöst. Zu dieser Lösung gibt man 7,0 ml einer 6%igen Gelatinelösung, 0,5 ml einer 8%igen Lösung des Netzmittels der Formel

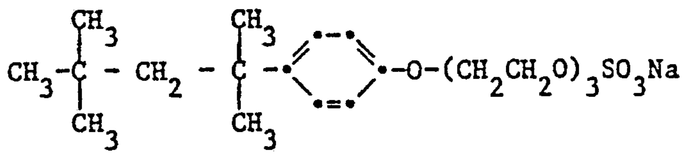

in Isopropanol/Wasser (3:4) und 0,5 ml Wasser und emulgiert mit Ultraschall bei einer Leistung von 100 Watt während 5 Minuten.

Zu 2,5 ml der so erhaltenen Emulsion gibt man 2,0 ml einer Silberbromid-Emulsion mit einem Silbergehalt von 6,0 g pro Liter, 0,7 ml einer 1%igen wässerigen Lösung des Härters der Formel

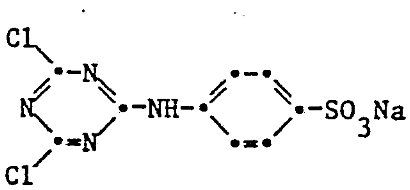

und 3,8 ml Wasser, stellt das Gemisch auf einen pH-Wert von 6,5 und vergiesst es auf ein auf eine Glasplatte aufgezogenes substriertes, kunststoffbeschichtetes, weisses Papier.

Nach dem Erstarren wird in einem Trockenschrank mit Umluft bei Raumtemperatur getrocknet.

Nach 7 Tagen werden auf 35×180 mm geschnittene Proben hinter einem Stufenkeil mit 3000 Lux·s belichtet und anschliessend im Ektaprint® 2-Prozess der Firma Kodak verarbeitet.

Die so erhaltenen Gelbkeile werden in einem Atlas Weather-Ometer mit einer 2500 W-Xenonlampe mit total 42 kJoule/cm² bestrahlt (eine Vergleichsprobe enthält kein Lichtschutzmittel). Der dabei eingetretene Farbdichteverlust wird bestimmt durch Messung der Farbdichte bei $\lambda_{max}$ mit einem Densitometer (TR 924 A der Fa. Macbeth®).

Die Ergebnisse sind in der nachfolgenden Tabelle aufgeführt.

| Stabilisator Verbindung Nr. | Prozentualer Farbdichteverlust |
|---|---|
| 5 | 24 |
| 13 | 19 |
| 14 | 20 |
| 26 | 23 |
| ohne Stab. | 36 |

**Patentansprüche**

1. Farbphotographisches Aufzeichnungsmaterial, das in mindestens einer lichtempfindlichen Silberhalogenid-Emulsionsschicht, einer Zwischenschicht, einer Bildempfangsschicht und/oder einer Schutzschicht als Stabilisator mindestens eine Verbindung enthält, die in ihrem Molekül mindestens einen Hydrochinonmonoether- oder Resorcinmonoether-Rest, dessen freie OH-Gruppe sterisch gehindert ist, und mindestens einen Polyalkylpiperidinrest enthalten.

2. Farbphotographisches Aufzeichnungsmaterial gemäss Anspruch 1, enthaltend als Stabilisator mindestens eine Verbindung der Formel I,

$$[A]_a Z [B]_b \qquad (I)$$

worin a und b unabhängig voneinander eine ganze Zahl von 1 bis 5 sind und (a+b) eine Zahl von 2 bis 6 ist,

A ein einwertiger Hydrochinonmonoether- oder Resorcinmonoether-Rest ist, dessen freie OH-Gruppen sterisch gehindert ist,

Z ein (a+b)-wertiger organischer Rest ist, der die Gruppen A und B verbindet, und

B ein einwertiger Polyalkylpiperidinrest der Formel II oder III ist,

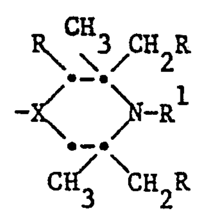

(II)

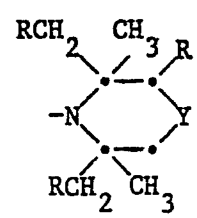

(III)

worin R Wasserstoff oder Methyl ist,

X eine Gruppe -CH oder einen 5- oder 6gliedrigen heterocyclischen Spiroring mit zwei O- oder N-Atomen bedeutet,

Y eine Gruppe $-CH_2-$ oder $-CH(R^2)-$ oder einen 5- oder 6-gliedrigen heterocyclischen Spiroring mit zwei O- oder N-Atomen bedeutet,

$R^1$ Hydroxyl, $C_1$-$C_{12}$-Alkyl, $C_3$-$C_6$-Alkenyl, $C_3$-$C_4$-Alkinyl, $C_7$-$C_{12}$-Phenylalkyl, Glycidyl, durch Halogen, -CN, $-COOR^3$ oder $-CON(R^4)(R^5)$ substituiertes $C_1$-$C_4$-Alkyl, eine Gruppe $-CO-R^6$, $-CO-OR^3$, $-CO-N(R^4)(R^5)$, $-CH_2-CH(R^7)-OR^8$, $-SO-R^9$,

$-SO_2-R^9$, $-OR^3$ oder $-OOC-R^6$ bedeutet, oder eine Gruppe $-Z^o-A$ bedeutet, worin $Z^o$ ein zweiwertiger organischer Rest ist, und im Falle, dass b = 1 ist, $R^1$ auch eine Gruppe der Formel

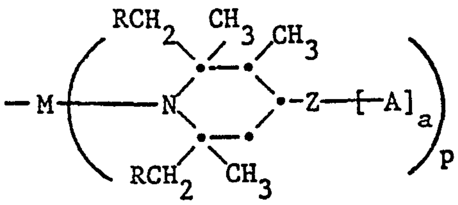

sein kann, worin p 1, 2 oder 3 ist und

M, wenn p = 1 ist, eine zweiwertige Gruppe ist und $C_2-C_{12}$-Alkylen, $C_4-C_8$-Alkenylen, Xylylen oder einen Rest der Formel $-CH_2C{\equiv}C-CH_2-$,

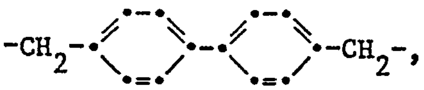

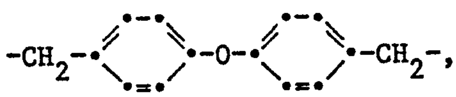

$-CH_2-COO-R^{10}-OOC-CH_2-$, $-CH_2-CH(OH)-CH_2-$, $-CH_2CH(OH)CH_2-D-CH_2CH(OH)CH_2-$, $-CH_2-CH(R^7)-OOC-R^{11}-COO-CH(R^7)-CH_2-$ oder $-CO-NH-G-NH-CO-$ darstellt, worin D einen zweiwertigen Rest der Formel $-O-R^{12}-O-$ oder $-OOC-R^{11}-COO-$ darstellt und G einen zweiwertigen aliphatischen, cycloaliphatischen, aromatischen oder aromatisch-aliphatischen Rest mit 6–15 C-Atomen bedeutet,

M, wenn p = 2 ist, einen dreiwertigen Rest der Formel

$T\text{-}[CH_2CH(OH)CH_2\text{-}]_3$ oder

$R^{13}\text{-}[COO-CH(R^7)-CH_2\text{-}]_3$

darstellt, worin T ein dreiwertiger Rest der Formeln

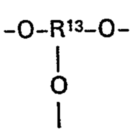 oder 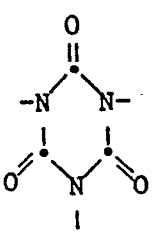 ist, und

M, wenn p = 3 ist, einen vierwertigen Rest der Formel

$Q\text{-}[CH_2CH(OH)CH_2\text{-}]_4$ oder

$R^{14}\text{-}[COO-CH(R^7)-CH_2\text{-}]_4$

darstellt, worin Q eine Gruppe der Formel

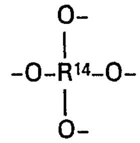 oder

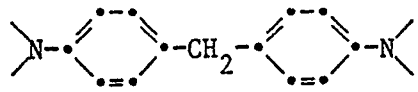 ist,

$R^2$ Hydroxyl, $-OR^{15}$, $-OCC-R^6$, $-OOC-N(R^4)(R^5)$, $-N(R^{16})-CO-R^6$ oder $-N(R^{16})-CO-N(R^4)(R^5)$ bedeutet, oder eine Gruppe $-Z^o-A$ bedeutet und im Falle, dass b = 1 ist, $R^2$ auch eine Gruppe der Formel

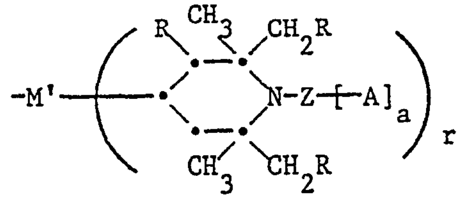

sein kann, worin r 1, 2 oder 3 ist und

M', wenn r = 1 ist, eine zweiwertige Gruppe der Formel $-OOC-R^{11}-COO-$, $-OOC-NH-G-NH-COO-$, $-N(R^{16})-CO-R^{11}-CO-N-(R^{16})-$ oder $-N(R^{17})-R^{18}-N(R(R^{17})-$, wenn r = 2 ist, eine dreiwertige Gruppe der Formel

$R^{19}[COO\text{-}]_3$ oder $R^{19}[CON(R^{16})\text{-}]_3$

und wenn r = 3 ist, eine vierwertige Gruppe der Formel

$R^{20}[COO\text{-}]_4$ oder $R^{20}[CON(R^{16})\text{-}]_3$ bedeutet

$R^3$ $C_1-C_{12}$-Alkyl, Benzyl oder Cyclohexyl bedeutet,

$R^4$ $C_1-C_{12}$-Alkyl, Allyl, Cyclohexyl, Benzyl oder Phenyl und

$R^5$ Wasserstoff, $C_1-C_8$-Alkyl oder Allyl bedeuten oder

$R^4$ und $R^5$ zusammen mit dem N-Atom einen 5- oder 6gliedrigen heterocyclischen Ring bilden,

$R^6$ Wasserstoff, $C_1-C_{12}$-Alkyl, $C_2-C_6$-Alkenyl, Chlormethyl, $C_5-C_{12}$-Cycloalkyl, $C_7-C_{12}$-Phenylalkyl, Phenyl, $C_7-C_{10}$-Alkylphenyl oder durch 1 oder 2 $C_1-C_4$-Alkylgruppen und eine Hydroxygruppe substituiertes Phenyl, Phenylmethyl oder Phenylethyl bedeutet,

$R^7$ Wasserstoff, $C_1-C_4$-Alkyl, $C_2-C_{13}$-Alkoxymethyl, Phenyl oder Phenoxymethyl bedeutet,

$R^8$ Wasserstoff, $C_1-C_{12}$-Alkyl, $-CO-R^6$ oder $-CO-N(R^4)(R^5)$ bedeutet,

$R^9$ $C_1-C_{12}$-Alkyl, Phenyl oder $C_7-C_{22}$-Alkylaryl bedeutet,

$R^{10}$ $C_2-C_{12}$-Alkylen, $C_4-C_8$-Oxaalkylen oder Cyclohexylen bedeutet,

$R^{11}$ eine direkte Bindung, $C_1-C_{12}$-Alkylen, $C_2-C_6$-

Alkenylen, $C_6$–$C_{12}$-Cycloalkylen oder -Cycloalkenylen oder $C_6$–$C_{12}$-Arylen bedeutet,

$R^{12}$ $C_2$–$C_{12}$-Alkylen, $C_6$–$C_{12}$-Cycloalkylen, $C_6$–$C_{12}$-Arylen oder -Phenylen-W-Phenylen- und W -O-, $-CH_2-$, $\geq C(CH_3)_2$ oder $-SO_2-$ bedeutet,

$R^{13}$ ein dreiwertiger aliphatischer Kohlenwasserstoffrest mit 3–10 Kohlenstoffatomen oder ein dreiwertiger aromatischer Kohlenwasserstoffrest mit 6–10 C-Atomen ist,

$R^{14}$ einen vierwertigen aliphatischen Kohlenwasserstoffrest mit 4–10 C-Atomen oder einen vierwertigen aromatischen Kohlenwasserstoffrest mit 6–12 C-Atomen bedeutet,

$R^{15}$ Wasserstoff, $C_1$–$C_{12}$-Alkyl, Allyl oder Benzyl. ist,

$R^{16}$ Wasserstoff, $C_1$–$C_{12}$-Alkyl, $C_5$–$C_{12}$-Cycloalkyl oder Benzyl ist,

$R^{17}$ Wasserstoff, $C_1$–$C_{12}$-Alkyl, Allyl, Benzyl, $C_2$–$C_{12}$-Alkanoyl oder Benzoyl bedeutet,

$R^{18}$ $C_2$–$C_{12}$-Alkylen, durch ein oder mehrere -O- oder $-N(R^{16})-$ unterbrochenes $C_4$–$C_{16}$-Alkylen oder $C_6$–$C_{12}$-Cycloalkylen bedeutet,

$R^{19}$ ein dreiwertiger aliphatischer Rest mit 3–10 C-Atomen oder ein dreiwertiger aromatischer Rest mit 6–10 C-Atomen oder die Gruppe $N(CH_2)_3$ und

$R^{20}$ ein vierwertiger aliphatischer Rest mit 4–10 C-Atomen oder ein vierwertiger aromatischer Rest mit 6–12 C-Atomen ist.

3. Farbphotographisches Aufzeichnungsmaterial gemäss Anspruch 2, enthaltend als Stabilisator eine Verbindung der Formel I, worin A ein sterisch gehinderter Hydrochinonmonoether-Rest ist.

4. Farbphotographisches Aufzeichnungsmaterial gemäss Anspruch 2, enthaltend als Stabilisator eine Verbindung der Formel I, worin B ein Polyalkylpiperidinrest der Formel II oder III ist, in der R Wasserstoff ist.

5. Farbphotographisches Aufzeichnungsmaterial gemäss Anspruch 3, enthaltend als Stabilisator eine Verbindung der Formel IV,

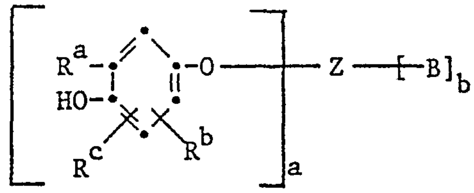 (IV)

worin a und b unabhängig voneinander eine Zahl von 1 bis 3 sind und (a+b) eine Zahl von 2 bis 4 ist,

$R^a$ ein einwertiger Kohlenwasserstoffrest ist, der die OH-Gruppe sterisch hindert,

$R^b$ Wasserstoff ist oder im Falle, dass a = 1 ist, auch ein Rest der Formel

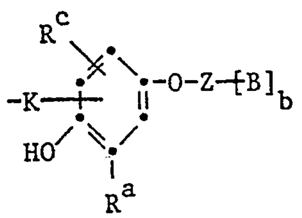

sein kann, worin K eine direkte Bindung oder eine zweiwertige Gruppe der Formel -S-, -S-S-, -SO-, $-SO_2$, $-CH_2SCH_2-$, $-CH_2OCH_2-$, $-CH(R^{21})-$ oder $-N(R^{22})-$ ist, wobei $R^{21}$ Wasserstoff, $C_1$–$C_{12}$-Alkyl oder durch -S- oder -O- unterbrochenes $C_3$–$C_{15}$-Alkyl und $R^{22}$ Wasserstoff, $C_1$–$C_{18}$-Alkyl oder Phenyl bedeutet,

$R^c$ Wasserstoff oder $C_1$–$C_8$-Alkyl ist, und Z und B die in Anspruch 2 gegebene Bedeutung haben.

6. Farbphotographisches Aufzeichnungsmaterial gemäss Anspruch 5, enthaltend als Stabilisator eine Verbindung der Formel IV, worin $R^a$ eine tertiäre Alkylgruppe mit 4–12 C-Atomen, Cyclohexyl, Phenyl oder α,α-Dimethylbenzyl ist und Z ein aliphatischer Rest ist, der b Ester- oder Amidgruppen enthält.

7. Farbphotographisches Aufzeichnungsmaterial gemäss Anspruch 6, enthaltend als Stabilisator eine Verbindung der Formel VII,

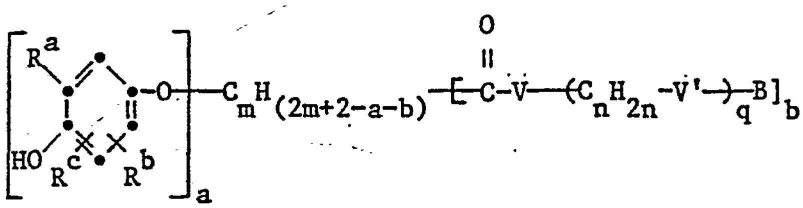 (VII)

worin

m eine Zahl von 1 bis 20, n eine Zahl von 1 bis 4 und q null oder 1 ist, V -O- oder $-N(R^{22})-$ und V' eine direkte Bindung, -O- oder $-N(R^{22})-$ bedeutet, B die in Anspruch 2 gegebene Bedeutung hat, a, b, $R^b$, $R^c$ und $R^{22}$ die in Anspruch 5 gegebene Bedeutung haben und $R^a$ die in Anspruch 6 gegebene Bedeutung hat, wobei im Falle, dass B ein Rest der Formel III ist, q 1 ist und V' eine direkte Bindung bedeutet.

8. Farbphotographisches Aufzeichnungsmaterial gemäss Anspruch 7, enthaltend als Stabilisator eine Verbindung der Formel VIIa oder VIIb,

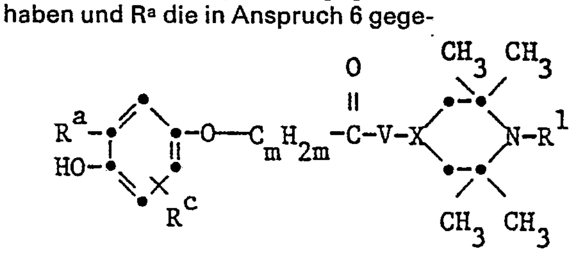 (VIIa)

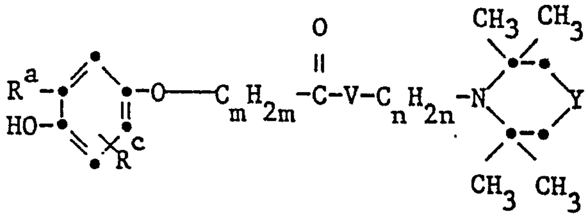

(VIIb)

worin m eine Zahl von 1 bis 6 ist, n, $R^a$, $R^c$ und V die in Anspruch 7 gegebenen Bedeutungen und X, Y und $R^1$ die in Anspruch 2 gegebene Bedeutung haben.

9. Farbphotographisches Aufzeichnungsmaterial gemäss Anspruch 3, enthaltend als Stabilisator eine Verbindung der Formel V

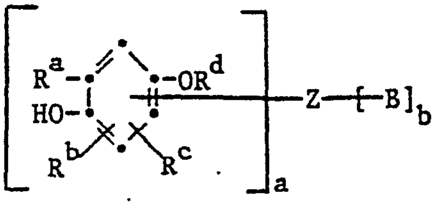

(V)

worin Z und B die in Anspruch 2 gegebenen Bedeutungen haben, a, b, $R^a$, $R^b$ und $R^c$ die in Anspruch 5 gegebenen Bedeutungen haben und $R^d$ $C_1$-$C_{20}$-Alkyl, $C_3$-$C_{15}$-Alkoxyalkyl, $C_5$-$C_{12}$-Cycloalkyl, $C_7$-$C_{13}$-Aralkyl, Phenyl oder ein Rest $-C_mH_{2m}-CO-V-C_nH_{2n}-B$ ist, worin m, n und V die in Anspruch 7 gegebene Bedeutung haben, oder $R^d$ zusammen mit einem orthoständigen $R^c$ und dem aromatischen Ring einen Cumaran- oder Chromanring bildet, und im Falle, dass a = 1 ist, $R^d$ auch ein Rest der Formel

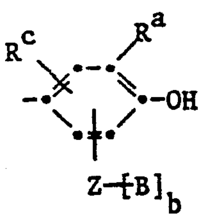

oder

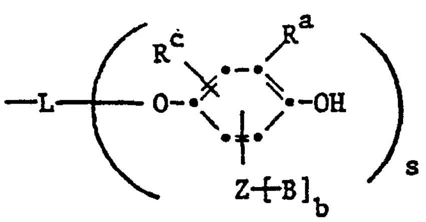

sein kann, worin s 1 oder 2 ist,

L, wenn s = 1 ist, $C_2$-$C_{12}$-Alkylen, das durch ein oder zwei O- oder S-Atome unterbrochen sein kann, $C_4$-$C_{10}$-Alkenylen, $C_4$-$C_8$-Alkinylen, $C_5$-$C_{12}$-Cycloalkylen, Xylylen oder einen Rest der Formeln

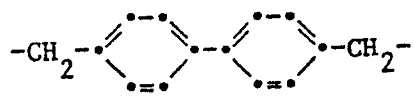

,

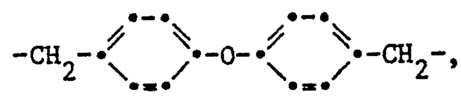

,

$-CH_2CH(OH)CH_2-O-R^{12}-O-CH_2CH(OH)CH_2-$ oder $-CH_2COO-R^{10}-OOCCH_2-$ bedeutet und $R^{10}$ und $R^{12}$ die in Anspruch 2 gegebene Bedeutung haben, und wenn s = 2 ist, L eine dreiwertige Gruppe der Formel

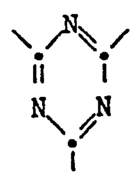

oder

$HOCH_2-C\text{—}(CH_2-)_3$

bedeutet.

10. Farbphotographisches Aufzeichnungsmaterial gemäss Anspruch 9, enthaltend als Stabilisator eine Verbindung der Formel V, worin $R^a$ eine tertiäre Alkylgruppe mit 4-12 C-Atomen, Cyclohexyl, Phenyl oder α-Dimethylbenzyl ist und Z ein aliphatischer Rest ist, der b Ester- oder Amidgruppen enthält.

11. Farbphotographisches Aufzeichnungsmaterial gemäss Anspruch 10, enthaltend als Stabilisator eine Verbindung der Formel VIIIa oder VIIIb

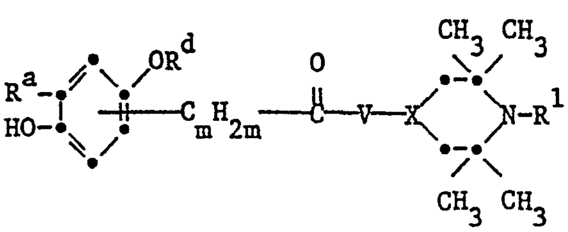

(VIIIa)

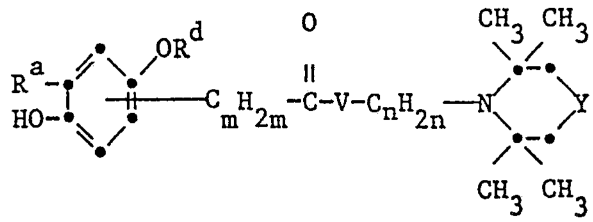

(VIIIb)

worin m eine Zahl von 1 bis 6 und n eine Zahl von 1 bis 4 ist, V -O- oder $-N(R^{22})-$ bedeutet, $R^a$ die in Anspruch 10 gegebene Bedeutung, $R^d$ die in Anspruch 9 gegebene Bedeutung, $R^{22}$ die in Anspruch 5 gegebene Bedeutung und X, $R^1$ und Y die in Anspruch 2 gegebene Bedeutung haben.

12. Farbphotographisches Aufzeichnungsmaterial gemäss Anspruch 3, enthaltend als Stabilisator eine Verbindung der Formel VI,

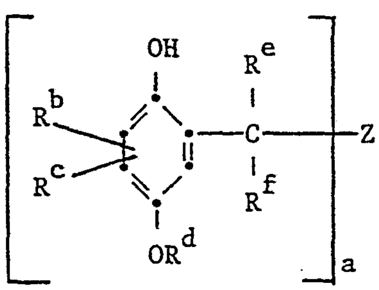

(VI)

worin a, b, $R^b$ und $R^c$ die in Anspruch 5 gegebenen Bedeutungen haben, und $R^b$ auch ein Rest der Formel IX sein kann,

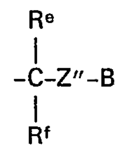

(IX)

$R^d$ die in Anspruch 9 gegebene Bedeutung hat, $R^e$ und $R^f$ $C_1$-$C_5$-Alkyl sind oder beide zusammen mit dem C-Atom, an das sie gebunden sind, einen Cycloalkan- oder Alkylcycloalkanring bilden oder eines von $R^e$ und $R^f$ zusammen mit dem C-Atom, an das sie gebunden sind, und mit dem Rest Z' oder einem Teil des Restes Z' einen Cycloalkan- oder Alkylcycloalkanring bilden, Z' einen (a+b)-wertigen organischen Rest bedeutet, Z'' einen 2-wertigen organischen Rest bedeutet und B die in Anspruch 2 gegebene Bedeutung hat.

13. Farbphotographisches Aufzeichnungsmaterial gemäss Anspruch 12, enthaltend als Stabilisator eine Verbindung der Formel VI, worin a 1 ist und Z' ein aliphatischer Rest ist, der b Ester- oder Amidgruppen enthält.

14. Farbphotographisches Aufzeichnungsmaterial gemäss Anspruch 13, enthaltend als Stabilisator eine Verbindung der Formel X,

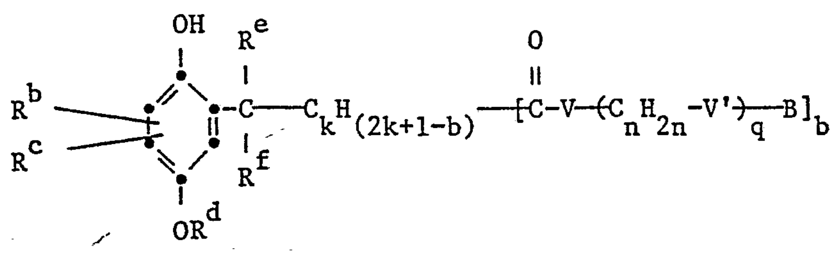

(X)

worin k null oder eine Zahl von 1 bis 20, n eine Zahl von 1 bis 4 und q null oder 1 ist, V -O- oder $-N(R^{22})-$ und V' eine direkte Bindung, -O- oder $-N(R^{22})-$ bedeutet und b, B, $R^b$, $R^c$, $R^d$, $R^e$ und $R^f$ die in Anspruch 12 gegebene Bedeutung haben, wobei im Falle, dass B ein Rest der Formel III ist, q 1 ist und V' eine direkte Bindung bedeutet.

15. Farbphotographisches Aufzeichnungsmaterial gemäss Anspruch 14, enthaltend als Stabilisator eine Verbindung der Formel XIa oder XIb,

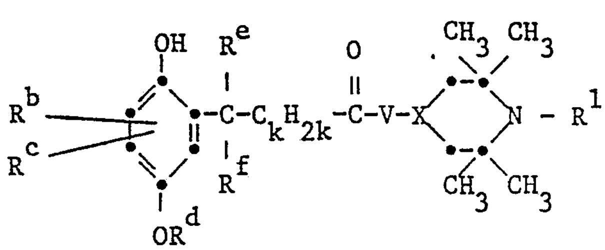

(XIa)

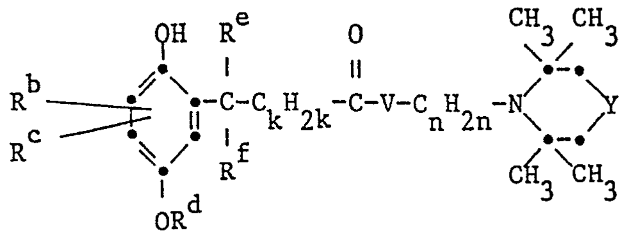
(XIb)

worin k null oder eine Zahl von 1 bis 6 ist, $R^b$ Wasserstoff oder eine Gruppe der Formel IXa oder IXb ist,

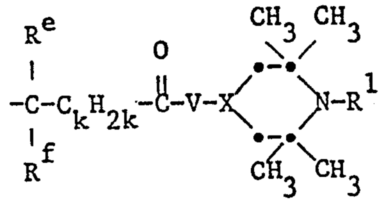
(IXa)

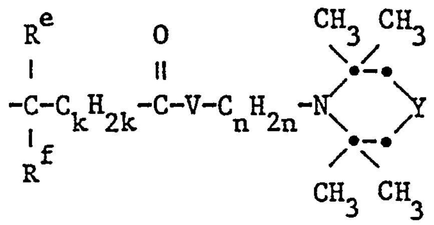
(IXb)

$R^e$ und $R^f$ Alkyl sind, n, $R^c$, $R^d$ und V die in Anspruch 14 gegebene Bedeutung haben und X, Y und $R^1$ die in Anspruch 2 gegebene Bedeutung haben.

16. Eine Verbindung der Formel I, wie sie in Anspruch 2 definiert ist.

17. Eine Verbindung der Formel IV, wie sie in Anspruch 5 definiert ist.

18. Eine Verbindung der Formel V, wie sie in Anspruch 9 definiert ist.

19. Eine Verbindung der Formel VI, wie sie in Anspruch 12 definiert ist.

**Claims**

1. A colour-photographic recording material which contains in at least one photosensitive silver halide emulsion layer, an interlayer, an image-receiving layer and/or a protective layer at least one compound, as stabiliser, which contains in its molecule at least one hydroquinone monoether or resorcinol monoether radical, the free OH group of which is sterically hindered, and at least one polyalkylpiperidine radical.

2. A colour-photographic recording material according to claim 1 containing, as stabiliser, at least one compound of the formula I

$$[A]_a-Z-[B]_b \qquad (I)$$

wherein a and b are each independently of the other an integer from 1 to 5 and (a+b) is a number from 2 to 6,

A is a monovalent hydroquinone monoether or resorcinol monoether radical, the free OH group of which is sterically hindered,

Z is an organic radical of valency (a+b) linking groups A and B, and

B is a monovalent polyalkylpiperidine radical of the formula II or III

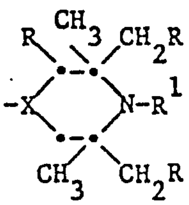
(II)

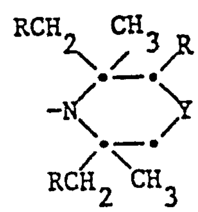
(III)

wherein R is hydrogen or methyl,

X is a group -CH or a 5- or 6-membered heterocyclic spiro ring with two O or N atoms,

Y is a $-CH_2-$ or $(CH(R^2)-$ group or a 5- or 6-membered heterocyclic spiro ring containing two O or N atoms,

$R^1$ is hydroxyl, $C_1$-$C_{12}$ alkyl, $C_3$-$C_6$ alkenyl, $C_3$-$C_4$ alkynyl, $C_7$-$C_{12}$ phenylalkyl, glycidyl, $C_1$-$C_4$ alkyl which is substituted by halogen, -CN, $-COOR^3$ or $-CON(R^4)R^5)$, or is a group $-CO-R^6$, $-CO-OR^3$, $-CO-N(R^4)(R^5)$, $-CH_2-CH(R^7)-OR^8$, $-SO-R^9$, $-SO_2-R^9$, $-OR^3$ or $-OOC-R^6$, or a group $-Z^o-A$, in which $Z^o$ is a divalent organic radical, and, if b is 1, $R^1$ can also be a group of the formula

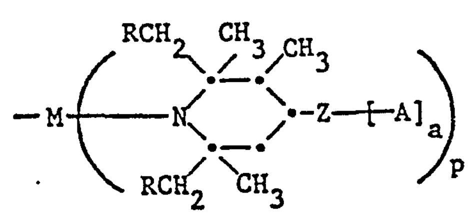

in which p is 1, 2 or 3, and

M if p is 1, is a divalent group and is $C_2$-$C_{12}$ alkylene, $C_4$-$C^8$ alkenylene, xylylene or a radical of the formula $-CH_2C{\equiv}C-CH_2-$

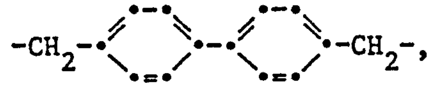

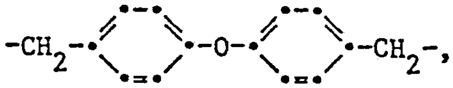

$-CH_2-COO-R^{10}-OOC-CH_2-$, $-CH_2-CH(OH)-CH_2-$, $-CHCH(OH)CH_2-D-CH_2CH(OH)CH_2-$, $-CH_2-CH(R^7)-OOC-R^{11}-COO-CH(R^7)-CH_2-$ or $-CO-NH-G-NH-CO-$, in which D is a divalent radical of the formula $-O-R^{12}-O-$ or $-OOC-R^{11}-COO-$, and G is a divalent aliphatic, cycloaliphatic, aromatic or aromaticaliphatic radical having 6–15 C atoms, or

M if p is 2, is a trivalent radical of the formula

$T\text{—}[CH_2CH(OH)CH_2]_3$ or

$R^{13}\text{—}[COO-CH(R^7)-CH_2]_3$

in which T is a trivalent radical of the formula

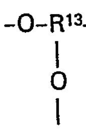 or 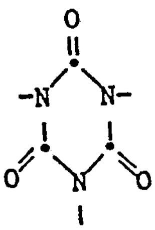

or

M if p is 3, is a quadrivalent radical of the formula

$Q\text{—}[CH_2CH(OH)CH_2]_4$ or

$R^{14}\text{—}[COO-CH(R^7)-CH_2]_4$

in which Q is a group of the formula

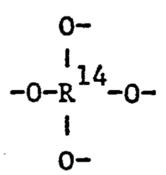 or

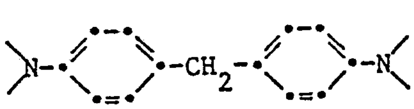

$R^2$ is a hydroxyl, $-OR^{15}$, $-OOC-R^6$, $-OOC-N(R^4)(R^5)$, $-N(R^{16})-CO-R^6$ or $-N(R^{16})-CO-N(R^4)(R^5)$, or is a group $-Z^o-A$, and, if b is 1, $R^2$ can also be a group of the formula

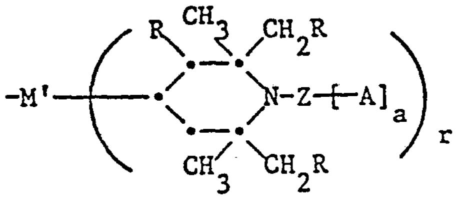

in which r is 1, 2 or 3, and

M′ if r is 1, is a divalent group of the formula $-OOC-R^{11}-COO$, $-OOC-NH-G-NH-COO-$, $-N(R^{16})-CO-R^{11}-CO-N-CR^{16})-$ or $-N(R^{17})-R^{18}-N(R^{17})-$, or, if r is 2, is a trivalent group of the formula

$R^{19}[COO-]_3$ or $R^{19}[CON(R^{16})-]_3$

or, if r is 3, is a quadrivalent group of the formula

$R^{20}[COO-]_4$ or $R^{20}[CON(R^{16})-]_3$

$R^3$ is $C_1$–$C_{12}$ alkyl, benzyl or cyclohexyl,

$R^4$ is $C_1$–$c_{12}$ alkyl, allyl, cyclohexyl, benzyl or phenyl, and

$R^5$ is hydrogen, $C_1$–$C_8$ alkyl or allyl, or

$R^4$ and $R^5$, together with the N atom, are a 5- or 6-membered heterocyclic ring,

$R^6$ is hydrogen, $C_1$–$C_{12}$ alkyl, $C_2$–$C_6$ alkenyl, chloromethyl, $C_5$–$C_{12}$ cycloalkyl, $C_7$–$C_{12}$ phenylalkyl, phenyl, $C_7$–$C_{10}$ alkylphenyl, or phenyl, phenylmethyl or phenethyl which is substituted by 1 or 2 $C_1$–$C_4$ alkyl groups and a hydroxyl group,

$R^7$ is hydrogen, $C_1$–$C_4$ alkyl, $C_2$–$C_{13}$ alkoxymethyl, phenyl or phenoxymethyl,

$R^8$ is hydrogen, $C_1$–$C_{12}$ alkyl, $-CO-R^6$ or $-CO-N(R^4)(R^5)$,

$R^9$ is $C_1$–$C_{12}$ alkyl, phenyl or $C_7$–$C_{22}$ alkylaryl,

$R^{10}$ is $C_2$–$C_{12}$ alkylene, $C_4$–$C_8$-oxaalkylene or cyclohexylene,

$R^{11}$ is a direct bond, $C_1$–$C_{12}$ alkylene, $C_2$–$C_6$ alkylene, $C_6$–$C_{12}$ cycloalkylene or $c_6$–$C_{12}$ cycloalkenylene or $C_6$–$C_{12}$ arylene,

$R^{12}$ is $C_2$–$C_{12}$ alkylene, $C_6$–$C_{12}$ cycloalkylene, $C_6$–$C_{12}$ arylene or -phenylene-W-phenylene- and W is $-O-$, $-CH_2-$, $>C(CH_3)_2$ or $-SO_2-$,

$R^{13}$ is a trivalent aliphatic hydrocarbon radical having 3–10 carbon atoms or a trivalent aromatic hydrocarbon radical having 6–10 C atoms,

$R^{14}$ is a quadrivalent aliphatic hydrocarbon radical having 4–10 C atoms or a quadrivalent aromatic hydrocarbon radical having 6–12 C atoms,

$R^{15}$ is hydrogen, $C_1$–$C_{12}$ alkyl, allyl or benzyl,

$R^{16}$ is hydrogen, $C_1$–$C_{12}$ alkyl, $C_5$–$C_{12}$ cycloalkyl or benzyl,

$R^{17}$ is hydrogen, $C_1$–$C_{12}$ alkyl, allyl, benzyl, $C_2$–$C_{12}$ alkanoyl or benzoyl,

$R^{18}$ is $C_2$–$C_{12}$ alkylene, $C_4$–$C_{16}$ alkylene which is interrupted by one or more $-O-$ or $-N(R^{16})-$, or $C_6$–$C_{12}$ cycloalkylene,

$R^{19}$ is a trivalent aliphatic radical having 3–10 C atoms, a trivalent aromatic radical having 6–10 C atoms or the group $N(CH_2)_3$, and

$R^{20}$ is a quadrivalent aliphatic radical having 4–10 C atoms or a quadrivalent aromatic radical having 6–12 C atoms.

3. A colour-photographic recording material according to claim 2 containing, as stabiliser, a compound of the formula I in which A is a sterically hindered hydroquinone monoether radical.

4. A colour-photographic recording material according to claim 2 containing, as stabiliser, a compound of the formula I in which B is a polyalkylpiperidine radical of the formula II or III, in which R is hydrogen.

5. A colour-photographic recording material according to claim 3 containing, as stabiliser, a compound of the formula IV

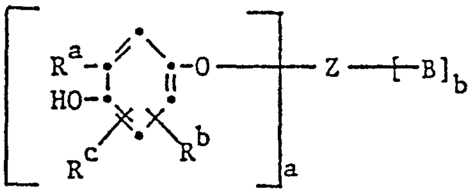
(IV)

in which a and b are each independently of the other a number from 1 to 3 and (a+b) is a number from 2 to 4,

$R^a$ is a monovalent hydrocarbon radical which sterically hinders the OH group,

$R^b$ is hydrogen or, if a is 1, can also be a radical of the formula

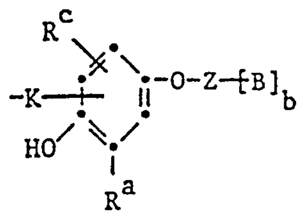

in which K is a direct bond or a divalent group of the formula -S-, -S-S-, -SO-, $-SO_2-$, $-CH_2SCH_2-$, $-CH_2OCH_2-$, $-CH(R^{21})-$ or $-N(R^{22})-$, in which $R^{21}$ is hydrogen, $C_1$–$C_{12}$ alkyl, or $C_3$–$C_{15}$ alkyl which is interrupted by -S- or -O- and $R^{22}$ is hydrogen, $C_1$–$C_{18}$ alkyl or phenyl, $R^c$ is hydrogen or $C_1$–$C_8$ alkyl, and Z and B are as defined in claim 2.

6. A colour-photographic recording material according to claim 5 containing, as stabiliser, a compound of the formula IV in which $R^a$ is a tertiary alkyl group having 4–12 C atoms, cyclohexyl, phenyl or α,α-dimethylbenzyl and Z is an aliphatic radical containing b ester or amide groups.

7. A colour-photographic recording material according to claim 6 containing, as stabiliser, a compound of the formula VII

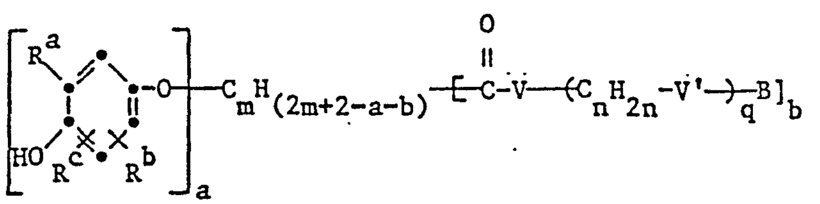
(VII)

in which m is a number from 1 to 20, n is a number from 1 to 4, q is zero or 1, V is -O- or $-N(R^{22})-$, V′ is a direct bond, -O- or $-N(R^{22})-$, B is as defined in claim 2, a, b, $R^b$, $R^c$ and $R^{22}$ are as defined in claim 5 and $R^a$ is as defined in claim 6, and, if B is a radical of the formula III, q is 1 and V′ is a direct bond.

8. A colour-photographic recording material according to claim 7 containing, as stabiliser, a compound of the formula VIIa or VIIb

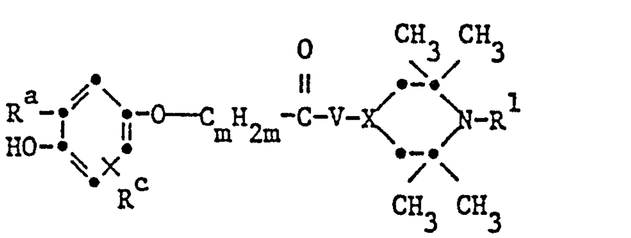
(VIIa)

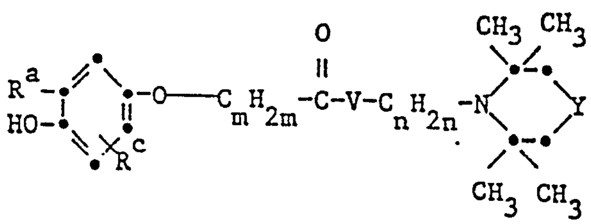
(VIIb)

in which m is a number from 1 to 6, n, $R^a$, $R^c$ and V are as defined in claim 7 and X, Y and $R^1$ are as defined in claim 2.

9. A colour-photographic recording material according to claim 3 containing, as stabiliser, a compound of the formula V

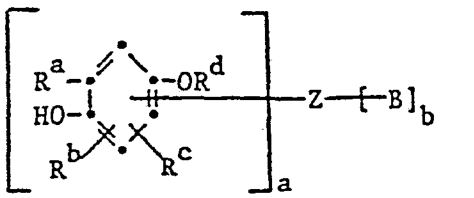 (V)

in which Z and B are as defined in claim 2, a, b, $R^a$, $R^b$ and $R^c$ are as defined in claim 5 and $R^d$ is $C_1$–$C_{20}$ alkyl, $C_3$–$C_{15}$ alkoxyalkyl, $C_5$–$C_{12}$ cycloalkyl, $C_7$–$C_{13}$ aralkyl, phenyl or a radical $-C_mH_{2m}-CO-V-C_nH_{2n}-B$, in which m, n and V are as defined in claim 7, or $R^d$, together with an $R^c$ in the ortho-position and the aromatic ring, is a coumaran or chroman ring, or, if a is 1, $R^d$ can also be a radical of the formula

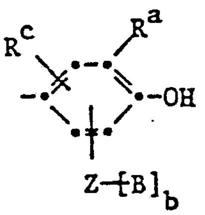 or

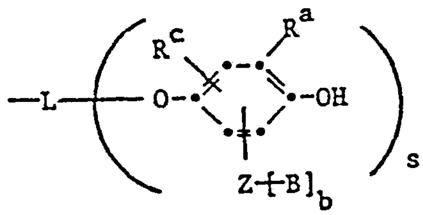

in which s is 1 or 2 and, if s is 1, L is $C_2$–$C_{12}$ alkylene, which can be interrupted by one or two O or S atoms, $C_4$–$C_{10}$ alkylene, $C_4$–$C_6$ alkynylene, $c_5$–$C_{12}$ cycloalkylene, xylylene or a radical of the formula

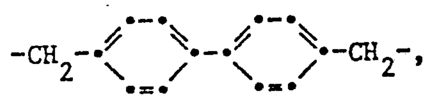

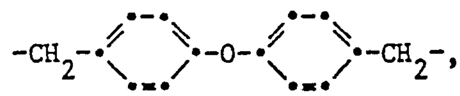

$-CH_2CH(OH)CH_2-$, $-CH_2CH(OH)CH_2-O-R^{12}-O-CH_2CH(OH)CH_2-$ or $-CH_2COO-R^{10}-OOCCH_2-$ and $R^{10}$ and $R^{12}$ are as defined in claim 2 or, if s is 2, L is a trivalent group of the formula

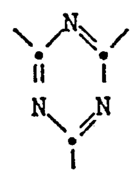 or

$HOCH_2-C-(CH_2-)_3$

10. A colour-photographic recording material according to claim 9 containing, as stabiliser, a compound of the formula V in which $R^a$ is a tertiary alkyl group having 4–12 C atoms, cyclohexyl, phenyl or α-dimethylbenzyl and Z is an aliphatic radical containing b ester or amide groups.

11. A colour-photographic recording material according to claim 10 containing, as stabiliser, a compound of the formula VIIIa or VIIIb

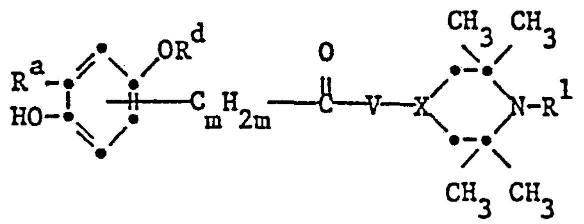 (VIIIa)

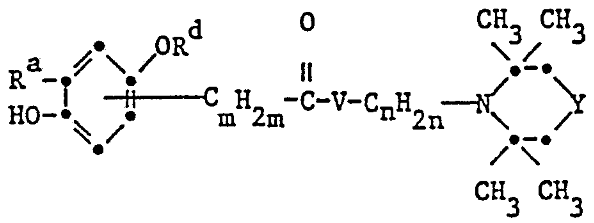 (VIIIb)

in which m is a number from 1 to 6, n is a number from 1 to 4, V is -O- or $-N(R^{22})-$, $R^a$ is as defined in claim 10, $R^d$ is as defined in claim 9, $R^{22}$ is as defined in claim 5 and X, $R^1$ and Y are as defined in claim 2.

12. A colour-photographic recording material

according to claim 3 containing, as stabiliser, a compound of the formula VI

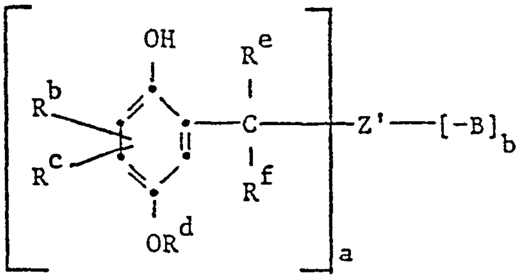

(VI)

in which a, b, $R^b$ and $R^c$ are as defined in claim 5, and $R^b_2$ can also be a radical of the formula IX

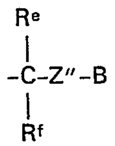

(IX)

$R^d$ is as defined in claim 9, $R^e$ and $R^f$ are each $C_1$–$C_5$ alkyl or, when taken together with the C atom to which they are attached, are a cycloalkane or alkylcycloalkane ring, or one of $R^e$ and $R^f$, together with the C atom to which it is attached and the radical Z′ or part of the radical Z′, is a cycloalkane or alkylcycloalkane ring, Z′ is an organic radical of valency (a+b), Z″ is a divalent organic radical and B is as defined in claim 2.

13. A colour-photographic recording material according to claim 12 containing, as stabiliser, a compound of the formula VI in which a is 1 and Z′ is an aliphatic radical containing b ester or amide groups.

14. A colour-photographic recording material according to claim 13 containing, as stabiliser, a compound of the formula X

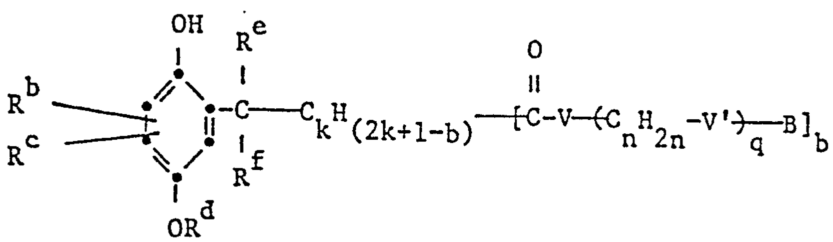

(X)

in which k is zero or a number from 1 to 20, n is a number from 1 to 4, q is zero or 1, V is -O- or $-N(R^{22})-$, V′ is a direct bond, -O- or $-N(R^{22})-$ and b, B, $R^b$, $R^c$, $R^d$, $R^e$ and $R^f$ are as defined in claim 12, and, if B is a radical of the formula III, q is 1 and V′ is a direct bond.

15. A recording material for colour photography according to claim 14 containing, as stabiliser, a compound of the formula XIa or XIb

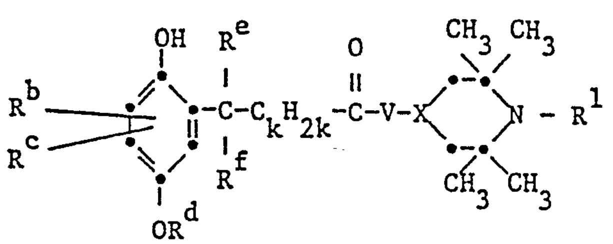

(XIa)

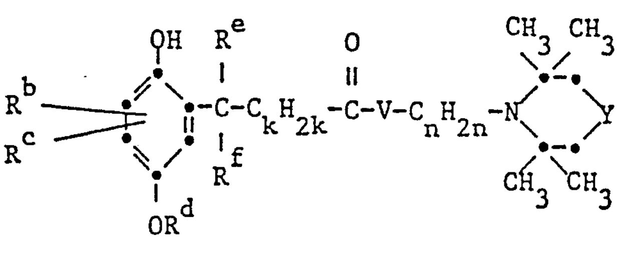

(XIb)

in which k is zero or a number from 1 to 6, $R^b$ is hydrogen or a group of the formula IXa or IXb

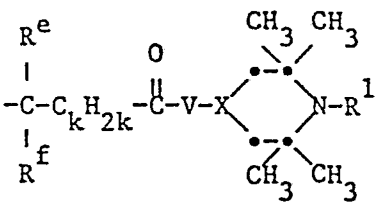
(IXa)

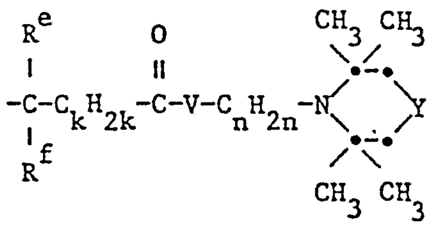
(IXb)

$R^e$ and $R^f$ are alkyl, n, $R^c$, $R^d$ and V are as defined in claim 14 and X, Y and $R^1$ are as defined in claim 2.

16. A compound of the formula I as defined in claim 2.

17. A compound of the formula IV as defined in claim 5.

18. A compound of the formula V as defined in claim 9.

19. A compound of the formula VI as defined in claim 12.

**Revendications**

1. Matière pour enregistrement photographique en couleurs qui, dans au moins une couche d'émulsion d'halogénure d'argent photosensible, une couche intermédiaire, une couche réceptrice d'images et/ou une couche protectrice, contient, comme stabilisant, au moins un composé dont la molécule renferme au moins un radical de monoéther d'hydroquinone ou de monoéther de résorcinol dont le radical hydroxy libre comporte un empêchement stérique, et au moins un radical polyalkyl-pipéridinique.

2. Matière pour enregistrement photographique en couleurs selon la revendication 1, caractérisée en ce qu'elle contient, comme stabilisant, au moins un composé répondant à la formule I:

$$[A]_a-Z-[B]_b \qquad (I)$$

dans laquelle:

a et b représentent chacun, indépendamment l'un de l'autre, un nombre entier de 1 à 5, la somme (a+b) désignant un nombre de 2 à 6,

A représente un radical univalent de monoéther d'hydroquinone ou de monoéther de résorcinol, le radical hydroxy libre de ce radical comportant un empêchement stérique,

Z représente un radical organique de valence égale à (a+b) qui unit les radicaux A et B et

B représente un radical univalent de polyalkylpipéridine répondant à l'une des formules II et III:

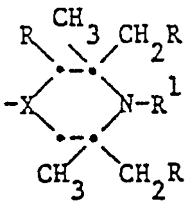
(II)

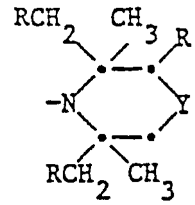
(III)

dans lesquelles:

R représente l'hydrogène ou un méthyle,

X représente un radical $-\underset{|}{C}H$ ou un cycle spirohétérocyclique contenant 5 ou 6 maillons dont deux atomes O ou N,

Y représente un radical $-CH_2-$ ou $-CH(R^2)-$ ou un cycle spirohétérocyclique à 5 ou 6 maillons qui contient deux atomes O ou N,

$R^1$ représente un hydroxy, un alkyle en $C_1$–$C_{12}$, un alcényle en $C_3$–$C_6$, un alcynyle en $C_3$ ou $C_4$, un phénylalkyle en $C_7$–$C_{12}$, un glycidyle, un alkyle en $C_1$–$C_4$ porteur d'un halogène ou d'un radical -CN, $-COOR^3$ ou $-CON(R^4)(R^5)$, ou un radical $-CO-R^6$, $-CO-OR^3$, $-CO-N(R^4)(R^5)$, $-CH_2-CH(R^7)-OR^8$, $-SO-R^9$, $-SO_2-R^9$, $-OR^3$ ou $-OOC-R^6$, ou représente un radical $-Z^o-A$ dont le symbole $Z^o$ représente un radical organique divalent, et, dans le cas où b est égal à 1, $R^1$ peut également représenter un radical répondant à la formule suivante:

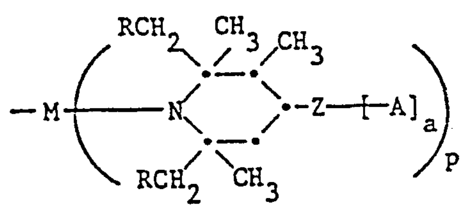

dans laquelle:

p est égal à 1, à 2 ou à 3 et

M représente:

– lorsque p est égal à 1 un radical divalent, en l'espèce un alkylène en $C_1$–$C_{12}$, un alcénylène en $C_4$–$C_8$, un xylylène ou un radical répondant à l'une des formules:

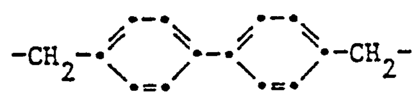

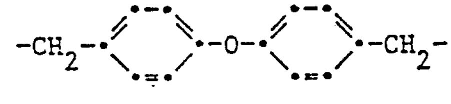

$-CH_2-COO-R^{10}-OOC-CH_2-$, $-CH_2-CH(OH)-CH_2-$, $-CH_2CH(OH)CH_2-D-CH_2CH(OH)CH_2-$, $-CH_2-CH(R^7)-OOC-R^{11}-COO-CH(R^7)-CH_2-$ et -CO-

NH-G-NH-CO, formules dans lesquelles D représente un radical divalent de formule $-O-R^{12}-O-$ ou de formule $-OOC-R^{11}-COO-$ et G représente un radical divalent aliphatique, cycloaliphatique, aromatique ou aromatique-aliphatique contenant de 6 à 15 atomes de carbone,

- lorsque p est égal à 2 un radical trivalent répondant à l'une des formules:

$T-[CH_2CH(OH)CH_2-]_3$ et

$R^{13}-[COO-CH(R^7)-CH_2-]_3$

où T représente un radical trivalent répondant à l'une des formules suivantes:

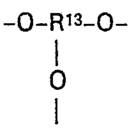
et
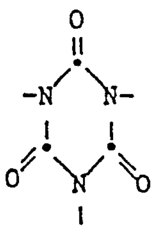

et

- lorsque p est égal à 3 un radical quadrivalent répondant à l'une des formules:

$Q-[-CH_2CH(OH)CH_2-]_4$ et

$R^{14}-[COO-CH(R^7)-CH_2-]_4$ ,

où Q représente un radical répondant à l'une des formules suivantes:

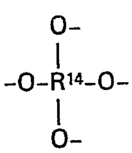
et

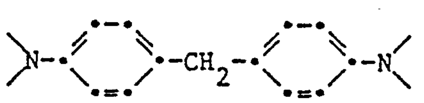
,

$R^2$ représente un radical hydroxy, $-OR^{15}$, $-OOC-R^6$, $-OOC-N(6R^4)(R^5)$, $-N(R^{16})-CO-R^6$ ou $-N(R^{16})-CO-N(R^4)(R^5)$, ou encore un radical $-Z^o-A$ et, dans le cas où b est égal à 1, $R^2$ peut également représenter un radical répondant à la formule:

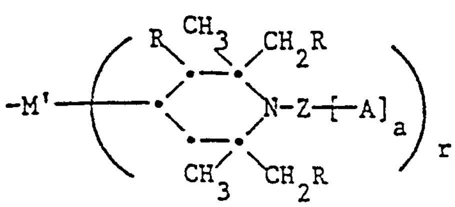

dans laquelle:

r est égal à 1, à 2 ou à 3 et M' représente:

- dans le cas où r est égal à 1 un radical bivalent répondant à l'une des formules $-OOC-R^{11}-COO-$, $-OOC-NH-G-NH-COO-$, $N(R^{16})-CO-R^{11}-CO-N(R^{16})-$ et $-N(R^{17})-R^{18}-N(R^{17})-$,

- dans le cas où r est égal à 2 un radical trivalent répondant à l'une des formules:

$R^{19}[COO-]_3$ et $R^{19}[CON(R^{16})-]_3$

et

- dans le cas où r est égal à 3 un radical quadrivalent répondant à l'une des formules:

$R^{20}[COO-]_4$ et $R^{20}[CON(R^{16})-]_3$ ,

$R^3$ représente un alkyle en $C_1-C_{12}$, un benzyle ou un cyclohexyle,

$R^4$ représente un alkyle en $C_1-C_{12}$, un allyle, un cyclohexyle, un benzyle ou un phényle,

$R^5$ représente l'hydrogène, un alkyle en $C_1-C_8$ ou un allyle,

$R^4$ et $R^5$ peuvent également former ensemble, et avec l'atome d'azote, un noyau hétérocyclique à 5 ou 6 maillons,

$R^6$ représente l'hydrogène, un alkyle en $C_1-C_{12}$, un alcényle en $C_2-C_6$, un chlorométhyle, un cycloalkyle en $C_5-C_{12}$, un phénylalkyle en $C_7-C_{12}$, un phényle, un alkylphényle en $C_7-C_{10}$ ou un radical phényle, phénylméthyle ou phényléthyle porteur d'un ou de deux radicaux alkyles en $C_1-C_4$ et d'un radical hydroxy,

$R^7$ représente l'hydrogène, un alkyle en $C_1-C_4$, un alcoxyméthyle en $C_2-C_{13}$, un phényle ou un phénoxyméthyle,

$R^8$ représente l'hydrogène, un alkyle en $C_1-C_{12}$, ou un radical $-CO-R^6$ ou $-CO-N(R^4)(R^5)$,

$R^9$ représente un alkyle en $C_1-C_{12}$, un phényle ou un alkylaryle en $C_7-C_{22}$,

$R^{10}$ représente un alkylène en $C_2-C_{12}$, un oxa-alkylène en $C_4-C_8$ ou un cyclohexylène,

$R^{11}$ représente une liaison directe, un alkylène en $C_1-C_{12}$, un alcénylène en $C_2-C_6$, un cycloalkylène en $C_6-C_{12}$, un cycloalcénylène en $C_6-C_{12}$ ou un arylène en $C_6-c_{12}$,

$R^{12}$ représente un alkylène en $C_2-C_{12}$, un cycloalkylène en $C_6-C_{12}$, un arylène en $C_6-C_{12}$ ou un radical phénylène-W-phénylène dont le symbole W représente un pont $-O-$, $-CH_2-$, $>C(CH_3)_2$ ou $-SO_2-$,

$R^{13}$ représente un radical hydrocarboné aliphatique trivalent contenant de 3 à 10 atomes de carbone ou un radical hydrocarboné aromatique trivalent contenant de 6 à 10 atomes de carbone,

$R^{14}$ représente un radical hydrocarboné aliphatique quadrivalent contenant de 4 à 10 atomes de carbone ou un radical hydrocarboné aromatique quadrivalent contenant de 6 à 12 atomes de carbone,

$R^{15}$ représente l'hydrogène, un alkyle en $C_1-C_{12}$, un allyle ou un benzyle,

$R^{16}$ représente l'hydrogène, un alkyle en $C_1$–$C_{12}$, un cycloalkyle en $C_5$–$C_{12}$ ou un benzyle,

$R^{17}$ représente l'hydrogène, un alkyle en $C_1$–$C_{12}$, un allyle, un benzyle, un alcanoyle en $C_2$–$C_{12}$ ou un benzoyle,

$R^{18}$ représente un alkylène en $C_2$–$C_{12}$, un alkylène en $C_4$–$C_{16}$ interrompu une ou plusieurs fois par -O- ou -N($R^{16}$)-, ou un cycloalkylène en $C_6$–$C_{12}$,

$R^{19}$ représente un radical aliphatique trivalent contenant de 3 à 10 atomes de carbone ou un radical aromatique trivalent contenant de 6 à 10 atomes de carbone, ou encore un radical $N(CH_2)_3$- et

$R^{20}$ représente un radical aliphatique quadrivalent contenant de 4 à 10 atomes de carbone ou un radical aromatique quadrivalent contenant de 6 à 12 atomes de carbone.

3. Matière pour enregistrement photographique en couleurs selon la revendication 2, matière qui contient, comme stabilisant, un composé de formule I dans lequel A représente un radical de monoéther d'hydroquinone à empêchement stérique.

4. Matière pour enregistrement photographique en couleurs selon la revendication 2, caractérisée en ce qu'elle contient, comme stabilisant, un composé de formule I dans lequel B représente un radical de polyalkyl-pipéridine répondant à l'une des formules II et III dont le symbole R représente l'hydrogène.

5. Matière pour enregistrement photographique en couleurs selon la revendication 3, qui contient, comme stabilisant, un composé répondant à la formule IV:

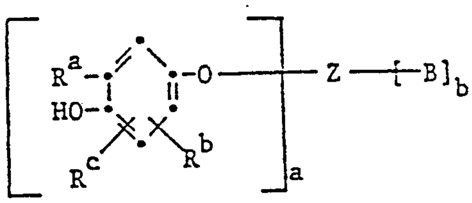

(IV)

dans laquelle:

a et b représentent chacun, indépendamment l'un de l'autre, un nombre de 1 à 3 et la somme (a+b) est un nombre de 2 à 4,

$R^a$ représente un radical hydrocarboné univalent qui crée un empêchement stérique pour le radical -OH,

$R^b$ représente l'hydrogène ou peut aussi représenter, dans le cas où a est égal à 1, un radical répondant à la formule:

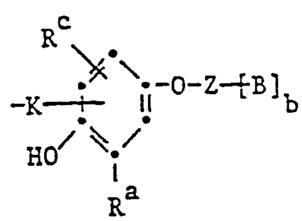

dans laquelle K représente une liaison directe ou l'un des radicaux divalents suivants: -S-, -S-S-, -SO-, $-SO_2-$, $-CH_2SCH_2-$, $-CH_2OCH_2-$, -CH($R^{21}$)- et -N($R^{22}$)-, où $R^{21}$ représente l'hydrogène, un alkyle en $C_1$–$C_{12}$ ou un alkyle en $C_3$–$C_{15}$ interrompu par -S- ou -O- et $R^{22}$ représente l'hydrogène, un alkyle en $C_1$–$C_{18}$ ou un phényle,

$R^c$ représente l'hydrogène ou un alkyle en $C_1$–$C_8$ et

Z et B ont les significations qui leur ont été données à la revendication 2.

6. Matière pour enregistrement photographique en couleurs selon la revendication 5, matière qui contient, comme stabilisant, un composé de formule IV dans lequel $R^a$ représente un alkyle tertiaire contenant de 4 à 12 atomes de carbone, un cyclohexyle, un phényle ou un α,α-diméthylbenzyle et Z représente un radical aliphatique contenant b radicaux esters ou amides.

7. Matière pour enregistrement photographique en couleurs selon la revendication 6, matière qui contient, comme stabilisant, un composé répondant à la formule VII:

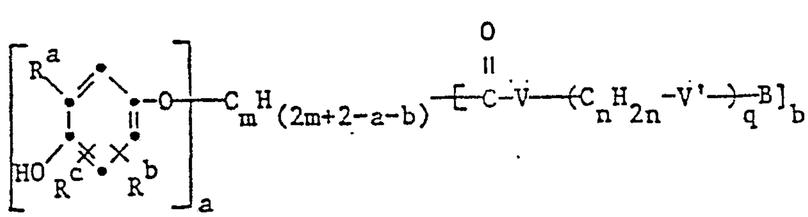

(VII)

dans laquelle m représente un nombre de 1 à 20, n un nombre de 1 à 4 et q un nombre égal à 0 ou à 1, V représente -O- ou $-N(R^{22})-$, V′ représente une liaison directe, -O- ou $-N(R^{22})-$, B a la signification qui lui a été donnée à la revendication 2, a, b, $R^b$, $R^c$ et $R^{22}$ ont les significations qui leur ont été données à la revendication 5 et $R^a$ a la signification qui lui a été donnée à la revendication 6, et, dans le cas où B représente un radical de formule III, q est égal à 1 et V′ représente une liaison directe.

8. Matière pour enregistrement photographique en couleurs selon la revendication 7, matière qui contient, comme stabilisant, un composé de répondant à l'une des formules VIIa et VIIb:

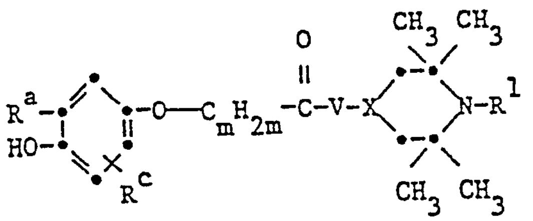

(VIIa)

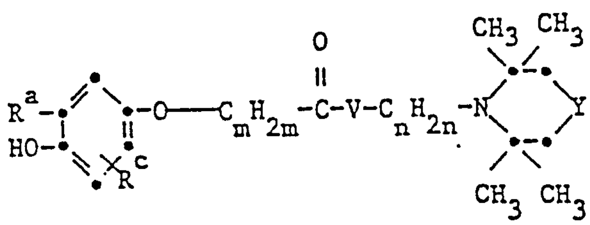

(VIIb)

dans lesquelles m désigne un nombre de 1 à 6, n, $R^a$, $R^c$ et V ont les significations donnéées à la revendication 7, et X, Y et $R^1$ les significations données à la revendication 2.

9. Matière pour enregistrement photographique en couleurs selon la revendication 3, matière qui contient, comme stabilisant, un composé répondant à la formule V:

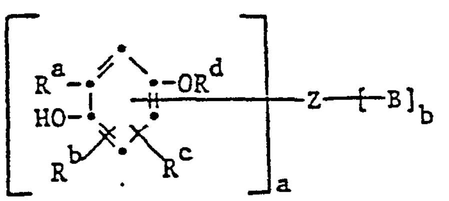

(V)

dans laquelle Z et B ont les significations données à la revendication 2, a, b, $R^a$, $R^b$ et $R^c$ ont les significations données à la revendication 5 et $R^d$ représente un alkyle en $C_1$–$C_{20}$, un alcoxyalkyle en $C_3$–$C_{15}$, un cycloalkyle en $C_5$–$C_{12}$, un aralkyle en $C_7$–$C_{13}$, un phényle ou un radical $-C_mH_{2m}-CO-V-C_nH_{2n}-B$ dans lequel m, n et V ont les significations données à la revendication 7, ou encore $R^d$ forme, avec le radical $R^c$ en ortho et le noyau aromatique, un cycle de coumaranne ou de chromanne, et, dans le cas où a est égal à 1, $R^d$ peut aussi représenter un radical répondant à l'une des formules:

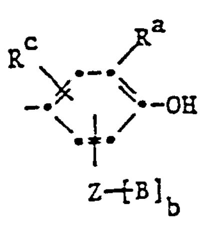

et

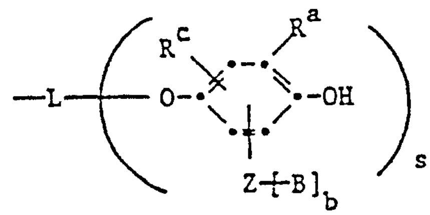

où s est égal à 1 ou à 2, L représente, lorsque s est égal à 1, un alkylène en $C_2$–$C_{12}$ éventuellement interrompu par un ou deux atomes O ou S, un alcénylène en $C_4$–$C_{10}$, un alcynylène en $C_4$–$C_6$, un cycloalkylène en $C_5$–$C_{12}$, un xylylène ou un radical répondant à l'une des formules:

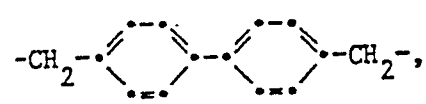,

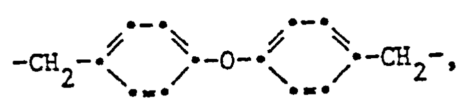,

$-CH_2CH(OH)CH_2$-, $-CH_2CH(OH)CH_2-O-R^{12}-O-CH_2CH(OH)CH_2-$ et $-CH_2COO-R^{10}-OOCCH_2-$ dans lesquelles $R^{10}$ et $R^{12}$ ont les significations données à la revendication 2, et, lorsque s est égal à 2, L représente un radical trivalent répondant à l'une des formules suivantes:

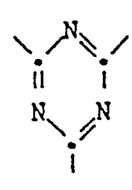

et $HOCH_2-C-(CH_2-)_3$ .

10. Matière pour enregistrement photographique en couleurs selon la revendication 9, matière qui contient, comme stabilisant, un composé de formule V dans lequel $R^a$ représente un alkyle tertiaire contenant de 4 à 12 atomes de carbone, un cyclohexyle, un phényle ou un α,α-diméthylbenzyle et Z un radical aliphatique contenant b radicaux esters ou amides.

11. Matière pour enregistrement photographique en couleurs selon la revendication 10, matière qui contient, comme stabilisant, un composé répondant à l'une des formules VIIIa et VIIIb:

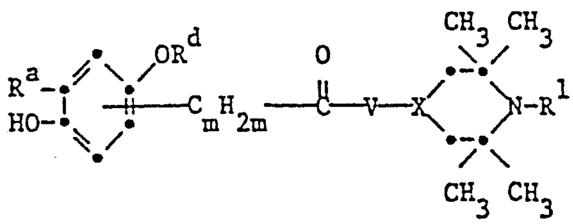

(VIIIa)

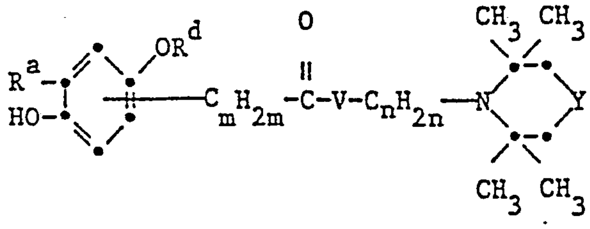

(VIIIb)

dans lesquelles m désigne un nombre de 1 à 6 et n un nombre de 1 à 4, V représente -O- ou $-N(R^{22})-$, $R^a$ a la signification donnée à la revendication 10, $R^d$ a la signification donnée à la revendication 9, $R^{22}$ a la signification donnée à la revendication 5, et X, $R^1$ et Y ont les significations données à la revendication 2.

12. Matière pour enregistrement photographique en couleurs selon la revendication 3, matière qui contient, comme stabilisant, un composé répondant à la formule VI:

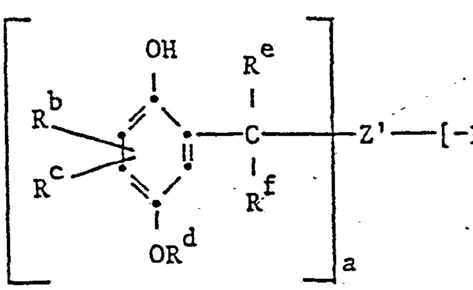

(VI)

dans laquelle a, b, $R^b$ et $R^c$ ont les significations données à la revendication 5, $R^b$ pouvant également représenter un radical de formule IX:

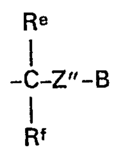

(IX)

$R^d$ a la signification donnée à la revendication 9, $R^e$ et $R^f$ représentent chacun un alkyle en $C_1$–$C_5$ ou forment ensemble et avec l'atome de carbone auquel ils sont liés un cycle de cycloalcane ou d'alkylcycloalcane, ou l'un des radicaux $R^e$ et $R^f$ forme, avec l'atome de carbone auquel il est lié et avec le radical Z′ ou une partie du radical Z′, un cycle de cycloalcane ou d'alkylcycloalcane, Z′ représente un radical organique de valence égale à (a+b), Z″ représente un radical organique bivalent et B a la signification donnée à la revendication 2.

13. Matière pour enregistrement photographique en couleurs selon la revendication 12, matière qui contient, comme stabilisant, un composé de formule VI dans lequel a est égal à 1 et Z′ représente un radical aliphatique contenant b radicaux esters ou amides.

14. Matière pour enregistrement photographique en couleurs selon la revendication 13, matière qui contient, comme stabilisant, un composé répondant à la formule X:

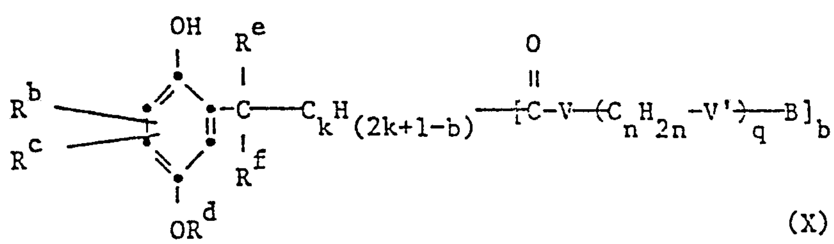

(X)

dans laquelle k est égal à 0 ou représente un nombre de 1 à 20, m désigne un nombre de 1-4 et q un nombre égal à 0 ou à 1, V représente -O- ou $-N(R^{22})-$, V′ représente une liaison directe, -O- ou $-N(R^{22})-$, et b, B, $R^b$, $R^c$, $R^d$, $R^e$ et $R^f$ ont les significations données à la revendication 12 et, dans le cas où B représente un radical de formule III, q est égal à 1 et V′ représente une liaison directe.

15. Matière pour enregistrement photographique en couleurs selon la revendication 14, matière qui contient, comme stabilisant, un composé répondant à l'une des formules XIa et XIb:

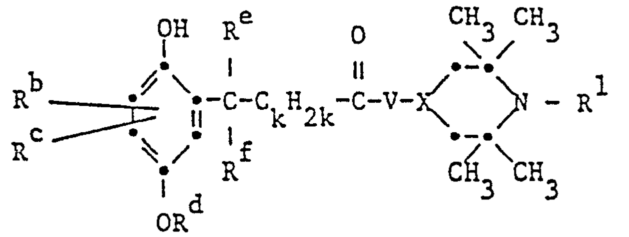

(XIa)

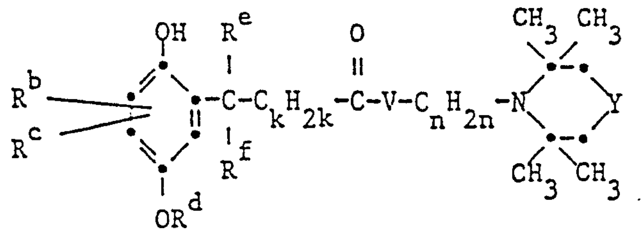

(XIb)

dans lesquelles k est égal à 0 ou désigne un nombre de 1 à 6, $R^b$ représente l'hydrogène ou un radical répondant à l'une des formules IXa et IXb:

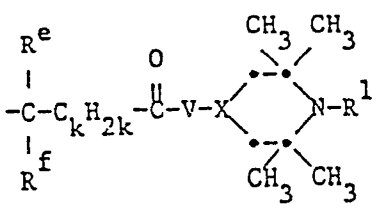

(IXa)

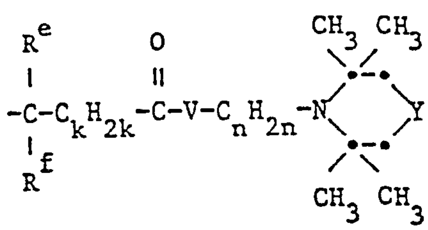

(IXb),

$R^e$ et $R^f$ représentent chacun un alkyle, n, $R^c$, $R^d$ et V ont les significations données à la revendication 14, et X, Y et $R^1$ ont les significations données à la revendication 2.

16. Composé de formule I tel que défini à la revendication 2.

17. Composé de formule IV tel que défini à la revendication 5.

18. Composé de formule V tel que défini à la revendication 9.

19. Composé de formule VI tel que défini à la revendication 12.